# EUROPÄISCHE PATENTSCHRIFT

(11) **EP 1 294 664 B1**
(45) Veröffentlichungstag und Bekanntmachung des Hinweises auf die Patenterteilung: **02.11.2005**
(21) Anmeldenummer: 01951635.0
(22) Anmeldetag: 25.06.2001
(51) Int. Cl.: C07C 31/125, C07C 29/16, C11D 1/72, C11D 1/14, C11D 1/34

(54) **ALKOHOLGEMISCHE MIT 13 UND 15 KOHLENSTOFFATOMEN UND DEREN VERWENDUNG IN DER HERSTELLUNG VON OBERFLÄCHENAKTIVEN SUBSTANZEN**
ALCOHOL MIXTURES HAVING 13 AND 15 CARBON ATOMS AND THE USE THEREOF IN THE PREPARATION OF SURFACE-ACTIVE SUBSTANCES
MELANGES D'ALCOOLS POSSEDANT 13 ET 15 ATOMES DE CARBONE, ET LEUR UTILISATION DANS LA PRODUCTION DE TENSIOACTIFS

(30) Priorität: 26.06.2000 DE 10031107
(43) Veröffentlichungstag der Anmeldung: 26.03.2003
(73) Patentinhaber: BASF Aktiengesellschaft, 67056 Ludwigshafen (DE)
(72) Erfinder: ZELLER, Edgar, 68165 Mannheim (DE); TROPSCH, Jürgen, 67354 Römerberg (DE)
(74) Vertreter: Kinzebach, Werner
(86) Internationale Anmeldenummer: PCT/EP2001/007220
(87) Internationale Veröffentlichungsnummer: WO 2002/000580

(56) Entgegenhaltungen:
- EP-A- 0 059 043
- EP-A- 0 627 439
- EP-A- 0 646 588
- WO-A-98/23566
- DE-A- 19 801 437
- US-A- 4 322 545
- US-A- 5 072 057
- US-A- 5 849 960

## Beschreibung

Die vorliegende Erfindung betrifft ein Alkoholgemisch, das im Wesentlichen Alkohole mit 13 und 15 Kohlenstoffatomen enthält, ein Verfahren zu seiner Herstellung, ein Verfahren zur Funktionalisierung dieser Alkoholgemische, die so erhaltenen funktionalisierten Alkoholgemische und deren Verwendung.

Es ist bekannt, Fettalkohole mit etwa 8 bis 20 Kohlenstoffatomen zur Herstellung von nichtionischen und anionischen Tensiden einzusetzen. Dazu werden die Alkohole einer entsprechenden Funktionalisierung, z. B. durch Alkoxilierung oder Glycosidierung unterworfen. Die resultierenden Alkoxilate können entweder direkt als nichtionische oberflächenaktive Substanzen eingesetzt oder durch eine weitere Funktionalisierung, z. B. durch Sulfatierung oder Phosphatierung, in anionische oberflächenaktive Substanzen überführt werden. Die anwendungstechnischen Eigenschaften dieser Tenside, z. B. deren Netzvermögen, Schaumbildung, Fettlösevermögen, biologische Abbaubarkeit etc., werden unter anderem durch die Kettenlänge und den Verzweigungsgrad des hydrophilen Kohlenwasserstoffrestes des eingesetzten Alkohols bestimmt. Alkohole, die sich gut für die Weiterverarbeitung zu wirksamen Tensiden eignen werden auch als Tensidalkohole bezeichnet.

In Kosswig/Stache, "Die Tenside", Carl Hanser Verlag, München, Wien, 1993, Kapitel 2.2 und 2.3, ist die Umsetzung von Fettalkoholen mit Alkylenoxiden zu den entsprechenden Fettalkoholalkoxilaten sowie deren Sulfatierung und Phosphatierung beschrieben.

Tensidalkohole sind sowohl aus nativen Quellen als auch auf synthetischem Weg, z. B. durch Hydrierung von nativen Fettsäuren oder durch Aufbau aus Edukten mit einer geringeren Zahl an Kohlenstoffatomen, erhältlich. Durch Aufbaureaktion von Ethen in Gegenwart von Triethylaluminium erhält man z. B. lineare primäre Alkohole, die sogenannten Ziegler-Alkohole. Nach dem ebenfalls bedeutsamen SHOP-Prozess (Shell higher olefine process) erhält man, ausgehend von Ethen, Olefinfraktionen mit einer für die Weiterverarbeitung zu Tensiden geeigneten Kohlenstoffanzahl. Die Funktionalisierung der Olefine zu den entsprechenden Alkoholen erfolgt dabei z. B. durch Hydroformylierung und Hydrierung. Eine Übersicht von Hydroformylierungsverfahren und geeigneten Katalysatoren findet sich in Beller et al. Journal of Molecular Catalysis A 104 (1995), S. 17-85. Dabei entstehen, auch beim Einsatz linearer Olefine, in der Regel Gemische aus geradkettigen und verzweigten primären Alkoholen.

Diese herkömmlichen Tensidalkohole sind in Bezug auf ihre anwendungstechnischen Eigenschaften, insbesondere die Bioabbaubarkeit, noch verbesserungswürdig.

Die WO-A 98/23566 beschreibt eine Zusammensetzung verzweigter primärer Alkohole und deren Sulfate, Alkoxilate, Alkoxysulfate und Carboxylate, die gute Kaltwasserwascheigenschaften und eine gute Bioabbaubarkeit aufweisen sollen. Die Zusammensetzungen weisen dabei Alkohole mit 8 bis 36 Kohlenstoffatomen und einen mittleren Verzweigungsgrad pro Molekül im Bereich von 0,7 bis 3,0 auf, wobei die Verzweigungen Methyl- und Ethyl-Verzweigungen umfassen. Auch diese Alkoholzusammensetzungen sind bezüglich ihrer anwendungstechnischer Eigenschaften noch verbesserungswürdig.

Der vorliegenden Erfindung liegt die Aufgabe zugrunde, ein neues Alkoholgemisch zur Verfügung zu stellen, das sich vorteilhaft für einen Einsatz als Tensidalkoholgemisch eignet. Dabei sollen die auf diesem Gemisch basierenden anionischen und/oder nichtionischen Tenside gute anwendungstechnische Eigenschaften, wie eine gute biologische Abbaubarkeit, ein geringes Schaumvermögen oder einen sich über einen möglichst geringen Konzentrationsbereich erstreckenden Gelbereich aufweisen. Der Erfindung liegt weiterhin die Aufgabe zugrunde, ein Verfahren zur Herstellung dieser Alkoholgemische zur Verfügung zu stellen.

Überraschenderweise wurde nun gefunden, dass die Aufgabe durch ein Alkoholgemisch gelöst wird, das im Wesentlichen Alkohole mit 13 und 15 Kohlenstoffatomen enthält, bei dem den Anteil aus der Summe un C₁₃- und C₁₅- Alkoholen bei mindestens 95 Gew.-% liegt und wobei mindestens 87 Gew.-% der Alkohole ausgewählt sind unter linearen, 2-Methyl-verzweigten und 2-Ethyl-verzweigten Alkoholen und wobei das Gemisch 40-60 Gew.-% lineare Alkohole, 30-40 Gew.-% 2-Methyl-verzweigte Alkohole und 2-7 Gew.-% 2-Ethylverzweigte Alkohole enthält.

Gegenstand der Erfindung ist daher ein Alkoholgemisch, das im Wesentlichen Alkohole mit 13 und 15 Kohlenstoffatomen enthält, wobei, bezogen auf den Gesamtalkoholgehalt, mindestens 87 Gew.-% der Alkohole ausgewählt sind unter linearen, 2-Methyl-verzweigten und 2-Ethyl-verzweigten Alkoholen.

Unter einem Alkoholgemisch wird vorliegend ein Gemisch verstanden, das wenigstens 2, bevorzugt wenigstens 3 verschiedene Alkohole aufweist.

Unter einem Alkoholgemisch, das im Wesentlichen Alkohole mit 13 und 15 Kohlenstoffatomen enthält, wird vorliegend ein Gemisch verstanden, bei dem der Anteil aus der Summe an C₁₃- und C₁₅-Alkoholen bei mindestens 95 Gew.-%, insbesondere mindestens 98 Gew.-%, liegt.

Bei dem erfindungsgemäßen Alkoholgemisch handelt es sich vorzugsweise um ein Gemisch primärer Alkohole.

Das Alkoholgemisch enthält 40 bis 60 Gew.-%, besonders bevorzugt 45 bis 55 Gew.-%, linearer Alkohole 30 bis 40 Gew.-%, besonders bevorzugt 33 bis 37 Gew.-%, 2-Methyl-verzweigte Alkohole und 2 bis 7 Gew.-%, besonders bevorzugt 4 bis 5,5 Gew.-%, 2-Ethyl-verzweigte Alkohole.

Die erfindungsgemäßen Alkoholgemische können zusätzlich noch weitere, von den zuvor genannten Alkoholen verschiedene, Alkohole aufweisen, wobei es sich z. B. um Alkohole mit längerkettigen Verzweigungen am C(2)-Atom, Alkohole mit Verzweigungen an einem anderen als dem C(2)-Atom oder Alkohole mit mehr als einer Verzweigungsstelle handelt. Diese werden im Folgenden unter dem Begriff "stärker verzweigte Alkohole" zusammengefasst.

Bevorzugt sind Alkoholgemische, bei denen das Verhältnis von Alkoholen mit 13 zu Alkoholen mit 15 Kohlenstoffatomen in einem Bereich von 90:10 bis 50:50 Gew.-%, bevorzugt 70:30 bis 60:40 Gew.-%, liegt.

Die Bestimmung der Zusammensetzung der erfindungsgemäßen Alkoholgemische kann nach üblichen, dem Fachmann bekannten Verfahren, wie z. B. mittels Gaschromatographie, erfolgen. Ein geeignetes gaschromatographisches Analyseverfahren umfasst die Auftrennung des Alkoholgemischs auf einer geeigneten Säule, isotherm oder mittels eines Temperaturprogramms, Detektion der Signale mittels eines geeigneten Detektors, wie z. B. eines Flammnionisations-, Wärmeleitfähigkeits- oder Elektroneneinfangsdetektors, Zuordnung der Signale zu den einzelnen Verbindungen, z. B. durch Vergleich mit den Retentionszeiten der entsprechenden reinen Alkohole (gegebenenfalls nach Zugabe eines internen Standards) und Integration der Signalflächen zur Ermittlung der Gewichtsmengenanteile der einzelnen Alkohole an dem Gemisch.

Die erfindungsgemäßen Alkoholgemische weisen vorzugsweise einen mittleren Verzweigungsgrad von höchstens 0,65, bevorzugt höchstens 0,5, auf.

Als Verzweigungsgrad ist die Zahl der Methylgruppen in einem Molekül des Alkohols abzüglich 1 definiert. Der mittlere Verzweigungsgrad ist der statistische Mittelwert der Verzweigungsgrade in einer Probe. Die mittlere Anzahl der Methylgruppen in den Molekülen einer Probe kann leicht mittels ¹H-NMR-Spektroskopie ermittelt werden. Hierzu wird die den Methylprotonen entsprechende Signalfläche durch drei dividiert und zu der durch zwei dividierten Signalfläche der Methylenprotonen der CH₂OH-Gruppe ins Verhältnis gesetzt. Die Bestimmung des mittleren Verzweigungsgrads kann auch rechnerisch aus der mittels Gaschromatographie ermittelten Zusammensetzung des Alkoholgemischs erfolgen.

Die erfindungsgemäßen Alkoholgemische eignen sich in vorteilhafter Weise für die Funktionalisierung zur Herstellung grenzflächenaktiver Gemische. Diese weisen vorteilhafterweise in der Regel bessere anwendungstechnische Eigenschaften, wie z. B. eine bessere biologische Abbaubarkeit, ein verringertes Schaumvermögen oder ein verbessertes Gelverhalten, auf als aus dem Stand der Technik bekannte Alkoholgemische.

Ein weiterer Gegenstand der Erfindung ist ein Verfahren zur Herstellung von Alkoholgemischen, wie zuvor beschrieben, wobei man:
a) ein Monoolefingemisch bereitstellt, das im Wesentlichen Olefine mit 12 und 14 Kohlenstoffatomen enthält und das lineare α-Olefine und einen Anteil von 5 bis 20 Gew.-% an davon verschiedenen Olefinen aufweist, und
b) das Monoolefingemisch durch Umsetzung mit Kohlenmonoxid und Wasserstoff in Gegenwart eines Rhodiumkatalysators hydroformyliert und hydriert.

### Schritt a)

Vorzugsweise wird in dem erfindungsgemäßen Verfahren ein großtechnisch anfallendes Monoolefingemisch eingesetzt.

Dazu zählen z. B. die durch gezielte Ethen-Oligomerisierung in Gegenwart von Alkylaluminiumkatalysatoren erhaltenen ziegler-Olefine. Dazu zählen weiterhin die durch Ethen-Oligomerisierung in Gegenwart verschiedener Katalysatorsysteme erhaltenen Olefine, z. B. die in Gegenwart von Alkylaluminiumchlorid/Titantetrachlorid-Katalysatoren erhaltenen und die in Gegenwart von Nickel-Phosphinkomplex-Katalysatoren nach dem Shell Higher Olefin Process (SHOP) erhaltenen Olefine. Geeignete technisch zugängige Olefingemische werden weiterhin bei der Paraffin-Dehydrierung entsprechender Erdölfraktionen, z. B. der sog. Petroleum- oder Dieselölfraktionen, erhalten. Zur Überführung von Paraffinen, vorwiegend von n-Paraffinen in Olefine, werden im Wesentlichen drei Verfahren eingesetzt:
- thermisches Cracken (Steamcracken),
- katalytisches Dehydrieren und
- chemisches Dehydrieren durch Chlorieren und Dehydrochlorieren.

Dabei führt das thermische Cracken überwiegend zu α-Olefinen, während die anderen Varianten Olefingemische ergeben, die im Allgemeinen auch Olefine mit innenständiger Doppelbindung aufweisen. Geeignete Olefingemische sind weiterhin die bei Metathese- bzw. Telomerisationsreaktionen erhaltenen Olefine. Dazu zählen z. B. die Olefine aus dem Philipps-Triolefin-Prozess, einem modifizierten SHOP-Prozess aus Ethylen-Oligomerisierung, Doppelbindungs-Isomerisierung und anschließender Metathese (Ethenolyse). Verfahren zur Herstellung geeigneter Olefine sind weiterhin die Herstellung von α,ω-Diolefinen durch ringöffnende Ethenolyse von Cycloolefinen, die Metathesepolymerisation von Cycloolefinen zu Polyalkenameren mit anschließender Ethenolyse etc. Allgemein wird bei der Ethenolyse eine hohe n-α-Olefinkonzentration erhalten.

Technisch zugängige Monoolefingemische aus Olefinen mit 12 und Olefinen mit 14 Kohlenstoffatomen werden auch als C₁₂-C₁₄-Olefine bezeichnet. Bevorzugt sind Gemische, bei denen das Verhältnis von Olefinen mit 12 zu Olefinen mit 14 Kohlenstoffatomen in einem Bereich von 90:10 bis 50:50 Gew.-%, bevorzugt 70:30 bis 60:40 Gew.-%, liegt. Besonders bevorzugt werden C₁₂-C₁₄-Olefine eingesetzt, bei denen das Olefin-Mischungsverhältnis im Bereich von etwa 2:1 liegt.

Vorteilhafterweise eignet sich das erfindungsgemäße Verfahren zur Herstellung von Alkoholgemischen aus technisch zugängigen Monoolefingemischen, die in der Regel nicht nur lineare α-Olefine, sondern auch Olefine mit innenliegenden Doppelbindungen sowie verzweigte Olefine enthalten. Dies führt zu einem wirtschaftlichen Vorteil des erfindungsgemäßen Verfahrens gegenüber Verfahren aus dem Stand der Technik, die auf den Einsatz von linearen α-olefinen angewiesen sind oder bei denen andere als lineare α-Olefine nicht in Wertprodukte umgesetzt werden. Geeignete technische Monoolefingemische weisen in der Regel bis zu 15 Gew.-% von α-Olefinen verschiedene Olefine auf. Dazu zählen neben Olefinen mit weiter innenliegenden Doppelbindungen auch Vinyliden-verzweigte Olefine, die eine Gruppe der Formel (-C(R^{a})=CH₂) aufweisen, worin R^{a} für einen Alkylrest, bevorzugt einen C₁-C₆-Alkylrest, insbesondere Methyl oder Ethyl steht. Derartige Vinyliden-verzweigte Olefinisomere werden z. B. bei der Dimerisierung von niedermolekularen Olefinschnitten oder beim Einbau höherer n-1-Olefine während der Ethenoligomerisierung im Ziegler-Prozess erhalten.

Bevorzugt wird in dem erfindungsgemäßen Verfahren ein Monoolefingemisch eingesetzt, das, bezogen auf den Gesamtolefingehalt, 85 bis 95 Gew.-% lineare α-Olefine, 1 bis 5 Gew.-% lineare interne Olefine, 5 bis 10 Gew.-% Vinyliden-verzweigte Olefine und gegebenenfalls bis zu 5 Gew.-% davon verschiedene sonstige Olefinisomere aufweist. Zu den sonstigen Olefinisomeren zählen dabei z. B. Isomere, die mehr als eine Verzweigungsstelle aufweisen und Isomere mit einer längerkettigen Vinylidenverzweigung.

Überraschenderweise führt das erfindungsgemäße Verfahren auch beim Einsatz großtechnisch zugängiger Monoolefingemische zu Alkoholgemischen, die bei einem Einsatz als Tensidalkoholgemische vorteilhafte anwendungstechnische Eigenschaften aufweisen.

### Schritt b)

Die Hydroformylierung in Schritt b) erfolgt unter Bedingungen, unter denen nicht nur die linearen α-Olefine, sondern im Wesentlichen alle Olefine des eingesetzten Monoolefingemischs hydroformyliert werden.

Erfindungsgemäß wird zur Hydroformylierung in Schritt b) ein Rhodium-Katalysator eingesetzt. Vorzugsweise handelt es sich bei den eingesetzten Rhodium-Katalysatoren nicht um Rhodium/Triarylphosphin-Katalysatoren, wie z. B. Rhodium/Triphenylphosphin-Katalysatoren, oder um Katalysatoren auf Basis von Liganden, die ähnliche sterische und elektronische Eigenschaften aufweisen, wie Triarylphosphine. Solche Katalysatoren eignen sich in der Regel nur zur Umsetzung von n-1-Olefinen bzw. des n-1-Olefinanteils von Olefingemischen.

Für die Hydroformylierung in Schritt b) werden Rhodium-Katalysatoren eingesetzt, die sich von üblichen, dem Fachmann bekannten Salzen oder Komplexen des Rhodiums ableiten, wie sie üblicherweise in Hydroformylierungsreaktionen verwendet werden. Dabei werden vorzugsweise Liganden eingesetzt, die den Katalysator befähigen, unter den Reaktionsbedingungen die Hydroformylierung sowohl von linearen α-Olefinen als auch von Olefinen mit innenständigen Doppelbindungen und/oder verzweigten Olefinen zu katalysieren. Bevorzugt weist der in Schritt b) eingesetzte Katalysator wenigstens einen Liganden auf, der ausgewählt ist unter zur Komplexbildung befähigten Verbindungen mit Carbonyl-, Carboxylat-, Hydrid-, Sulfat-, Nitrat-, stickstoffhaltigen und/oder phosphorhaltigen Gruppen, wobei die phosphorhaltige Gruppe höchstens einen Arylrest in Einfachbindung zum Phosphoratom aufweist.

Geeignete Rhodium-Katalysatoren bzw. -Katalysatorvorstufen sind Rhodium(II)- und Rhodium(III)salze wie Rhodium(III)chlorid, Rhodium(III)nitrat, Rhodium(III)sulfat, Kalium-Rhodiumsulfat (Rhodiumalaun), Rhodium(II)- bzw. Rhodium(III)carboxylat, vorzugsweise Rhodium(II)- und Rhodium(III)acetat, Rhodium(II)- und Rhodium(III)ethylhexanoat, Rhodium(III)oxid, Salze der Rhodium(III)säure und Trisammoniumhexachlororhodat(III).

Weiterhin eignen sich Rhodiumkomplexe der allgemeinen Formel RhXₘL¹L²(L³)ₙ, worin X für Halogenid, vorzugsweise Chlorid oder Bromid, Alkyl- oder Arylcarboxylat, Acetylacetonat, Aryl- oder Alkylsulfonat, insbesondere Phenylsulfonat und Toluolsulfonat, Hydrid oder das Diphenyltriazin-Anion, L¹, L², L³ unabhängig voneinander für CO, Olefine, Cycloolefine, vorzugsweise Cyclooctadien (COD) stehen. X steht bevorzugt für Hydrid, Chlorid, Bromid, Acetat, Tosylat, Acetylacetonat oder das Diphenyltriazin-Anion, insbesondere für Hydrid, Chlorid oder Acetat.

Nach einer ersten bevorzugten Ausführungsform wird in dem erfindungsgemäßen Verfahren ein Rhodium-Katalysator eingesetzt, der wenigstens eine Verbindung mit wenigstens einem unverbrückten Phosphacyclohexan- und/oder Phosphacyclohexen-Strukturelement als Liganden aufweist.

Vorzugsweise weist der Katalysator als Liganden wenigstens ein Phosphacyclohexan der allgemeinen Formeln I und II 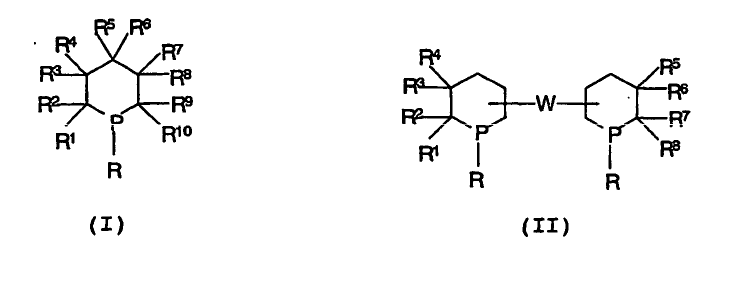 auf, mit der Bedeutung
- R: Wasserstoff, C1-100-Alkyl, Cycloalkyl, Heterocycloalkyl, C₇₋₂₀-Aralkyl, C₇₋₂₀-Alkaryl, C₆₋₁₂-Aryl, Hetaryl, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR', W'((CH₂)₄O)ₓR' oder W'COR',
wobei in der Formel II die Reste R auch anstelle von oder zusätzlich zu der Gruppe W zusammen eine Brücke mit 1 bis 20 Kohlenstoffatomen bilden können, die Bestandteil einer cyclischen oder aromatischen Gruppe sein kann und durch Heteroatome unterbrochen sein kann,
wobei in der Formel II die Reste R auch anstelle von oder zusätzlich zu der Gruppe W zusammen für eine Polyoxyalkylen- oder Polyalkylenimin-Brücke mit mindestens 21 Kohlenstoffatomen stehen können,
- R1 bis R¹⁰: unabhängig voneinander Wasserstoff, C1-20-Alkyl, C₇₋₂₀-Aralkyl, C₇₋₂₀-Alkaryl, C₆₋₁₂-Aryl, wobei eines oder mehrere Kohlenstoffatome durch Heteroatome ersetzt sein können, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR', W'((CH₂)₄O)ₓR', W'Halogen, W'NO₂, W'COR' oder W'CN,
wobei in den Resten R und R¹ bis R¹⁰ ein oder mehrere Wasserstoffatome durch Fluor ersetzt sein können,
- W und W': unabhängig voneinander Einfachbindungen oder Brücken mit 1 bis 20 Kohlenstoffatomen, die Bestandteil einer cyclischen oder aromatischen Gruppe sein können und durch Heteroatome unterbrochen sein können,
wobei W auch für eine Polyoxyalkylen- oder Polyalkylenimin-Brücke mit mindestens 21 Kohlenstoffatomen stehen kann,
- R': Wasserstoff, C1-20-Alkyl, Carbonylalkyl, Cycloalkyl oder Aryl,
- M⁺: Kationäquivalent,
- X⁻: Anionäquivalent,
- x: Zahl von 1 bis 240,
wobei zwei geminale Reste R1 bis R¹⁰ eine Oxo-Gruppe bilden können und einer oder mehrere der Reste R und R¹ bis R¹⁰ eine zusätzliche, zur Koordination befähigte dreibindige Phosphor- oder Stickstoffgruppierung aufweisen können,
wobei jeweils zwei vicinale Reste zu anellierten aliphatischen oder aromatischen Ringen verbunden sein können,
wobei zwei vicinale Reste R¹ bis R¹⁰ auch für eine chemische Bindung stehen können,
wobei in den Verbindungen der allgemeinen Formel II auch zwei oder mehr Brücken W vorliegen können und wobei die nicht an die Brücke(n) W gebundenen Atome der Phosphacyclohexanringe auch wie für R¹ bis R¹⁰ definiert substituiert sein können,
wobei in den Verbindungen der Formel I auch einer der Reste R oder R¹ bis R¹⁰ und in den Verbindungen der Formel II auch einer der Reste R oder R¹ bis R⁸ oder die beiden Reste R gemeinsam oder eine Gruppe W auch für einen Polymerrest mit einem zahlenmittleren Molekulargewicht im Bereich von 500 bis 50000 stehen kann, aufgebaut aus Wiederholungseinheiten, die sich von Monomeren ableiten, die ausgewählt sind unter Mono- und Diolefinen, Vinylaromaten, Estern α,β-ethylenisch ungesättigter Mono- und Dicarbonsäuren mit C₁-C₃₀-Alkanolen, N-Vinylamiden, N-Vinyllactamen, unter Ringöffnung polymerisierbaren heterocyclischen Verbindungen und Mischungen davon.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₁₀₀-Alkyl-, bevorzugterweise C₁-C₂₀-Alkyl- und besonders bevorzugt C₁-C₁₀-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl, Nonyl, Decyl.

Substituierte Alkylreste weisen vorzugsweise 1, 2, 3, 4 oder 5, insbesondere 1, 2 oder 3 Substituenten auf. Diese sind beispielsweise ausgewählt unter Cycloalkyl, Aryl, Hetaryl, Halogen, NE¹E², (NE¹E²E³)⁺, Carboxyl, Carboxylat, -SO₃H und Sulfonat.

Bei der Cycloalkylgruppe handelt es sich vorzugsweise um eine C₅-C₇-Cycloalkylgruppe, wie Cyclopentyl, Cyclohexyl oder Cycloheptyl.

Bei der Heterocycloalkylgruppe handelt es sich vorzugsweise um eine C₅₋₇-Heterocycloalkylgruppe. Bevorzugt weist die Heterocycloalkylengruppe 1, 2, 3 oder 4 gegebenenfalls substituierte Heteroatome auf. Dazu zählen beispielsweise Pyrrolidin, Tetrahydrofuran, Pyrazolidin, Imidazolidin, Piperidin, Piperazin und Morpholin.

Wenn die Cycloalkylgruppe oder Heterocycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, Xylyl, Mesityl, Naphthyl, Anthracenyl, Phenanthrenyl, Naphthacenyl und insbesondere für Phenyl, Naphthyl oder Xylyl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, -SO₃H, Sulfonat, NE¹E², Alkylen-NE¹E², Nitro, Cyano oder Halogen auf.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy, Carboxyl, Carboxylat, -SO₃H, Sulfonat, NE¹E², Alkylen-NE¹E², Trifluormethyl oder Halogen auf.

Die obigen Ausführungen zu Alkyl-, Cycloalkyl- und Arylresten gelten entsprechend für Alkoxy-, Cycloalkyloxy- und Aryloxyreste.

E¹, E² und E³ sind vorzugsweise unabhängig ausgewählt unter Wasserstoff, Alkyl und Cycloalkyl. Die Reste NE¹E² stehen vorzugsweise für N,N-Dimethyl, N,N-Diethyl, N,N-Dipropyl, N,N-Diisopropyl, N,N-Di-n-butyl, N,N-Di-tert.-butyl, N,N-Dicyclohexyl oder N,N-Diphenyl.

Heteroatom steht vorzugsweise für ein Sauerstoff-, Schwefel-, zweifach substituiertes Silicium- oder einfach substituiertes Stickstoffatom, wobei die Substituenten unabhängig voneinander für Wasserstoff, Alkyl, Cycloalkyl, Heterocycloalkyl, Aryl, Hetaryl, Alkoxy, Cycloalkoxy oder Aryloxy stehen.

Halogen steht für Fluor, Chlor, Brom und Iod, bevorzugt für Fluor, Chlor und Brom.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol.

Unter Polybutadienen werden im Rahmen dieser Erfindung immer auch deren teilweise oder vollständigen Hydrierungsprodukte verstanden.

Polyoxyalkylen steht vorzugsweise für Verbindungen mit Wiederholungseinheiten, die ausgewählt sind unter -(CH₂CH₂O)-ₓ₁, (̵CH(CH₃)CH₂O)̵ₓ₂ und (̵(CH₂)₄O)̵ₓ₃, wobei x₂, x₂ und x₃ unabhängig voneinander für eine ganze Zahl von 0 bis 240, vorzugsweise 0 bis 100, stehen. Die Summe aus x₁, x₂ und x₃ steht für eine ganze Zahl von 1 bis 240, vorzugsweise 2 bis 100. In Polyoxyalkylenen, die zwei oder drei verschiedenartige Wiederholungseinheiten aufweisen, ist die Reihenfolge beliebig, d. h. es kann sich um statistisch verteilte, alternierende oder blockförmige Wiederholungseinheiten handeln. Das zuvor für die Polyoxyalkylene Gesagte gilt analog für Polyalkylenimine, wobei das Sauerstoffatom jeweils durch eine Gruppe *NR*ⁱ ersetzt ist, worin Rⁱ für Wasserstoff oder C₁₋₄-Alkyl steht.

M⁺ steht für ein Kationäquivalent, d. h. für ein einwertiges Kation oder den einer positiven Einfachladung entsprechenden Anteil eines mehrwertigen Kations. Vorzugsweise steht M⁺ für ein Alkalimetallkation, wie z. B. Li⁺, Na⁺ oder K⁺ oder für ein Erdalkalimetallkation, für NH₄⁺ oder eine quartäre Ammonium-Verbindung, wie sie durch Protonierung oder Quarternierung von Aminen erhältlich ist. Bevorzugt handelt es sich um Alkalimetallkationen, insbesondere um Natrium- oder Kaliumionen.

X⁻ steht für ein Anionäquivalent, d. h. für ein einwertiges Anion oder den einer negativen Einfachladung entsprechenden Anteil eines mehrwertigen Anions. Vorzugsweise steht X- für ein Carbonat, Carboxylat oder Halogenid, besonders bevorzugt für Cl⁻ und Br⁻.

Die Werte für x stehen für eine ganze Zahl von 1 bis 240, vorzugsweise für eine ganze Zahl von 1 bis 100, insbesondere 1 bis 50, speziell 3 bis 40.

Wenn der Rest R für einen substituierten C₁₋₁₀₀-Alkylrest steht, so kann dieser z. B. ein- oder mehrfach durch wie für R¹ bis R¹⁰ angegebene Reste substituiert sein.

Bei den Resten R kann eines oder können mehrere der im Rest enthaltenen Kohlenstoffatome durch Heteroatome ersetzt sein.

Der Rest R ist vorzugsweise ausgewählt aus Phenyl- oder C₁₋₁₂-Alkylresten, wobei es sich um lineare, verzweigte oder cyclische Alkylreste handeln kann, die auch Sauerstoffatome als Heteroatome in der Kette enthalten können, z. B. in Form von Alkylenoxid-, insbesondere Ethylenoxideinheiten, die endständig alkyliert sein können.

Bevorzugt steht der Rest R für einen C₂₋₁₄-Alkylrest, insbesondere für Propyl, n-Butyl, sek.-Butyl, tert.-Butyl, Pentyl, Hexyl, Heptyl, Octyl, 2-Ethylhexyl oder Dodecyl.

Bevorzugt steht der Rest R weiterhin für einen C₅₋₈-Cycloalkylrest, insbesondere Cyclohexyl.

Bevorzugt steht der Rest R weiterhin für einen Polyoxyalkylen- oder Polyalkyleniminrest. Geeignete Polyoxyalkylene leiten sich z. B. von Formaldehyd (Polyoxymethylene), cyclischen Ethern wie Tetrahydrofuran, Alkylenoxiden mit 2 bis 4 Kohlenstoffatomen im Alkylrest und Kombinationen davon ab. Geeignete Alkylenoxide sind beispielsweise Ethylenoxid, 1,2-Propylenoxid, Epichlorhydrin, 1,2- und 2,3-Butylenoxid. Geeignete Polyalkylenimine leiten sich z. B. von Aziridinen (Alkyleniminen) der Formel 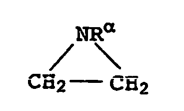 ab, worin R^{α} für Wasserstoff oder Alkyl steht. Das zahlenmittlere Molekulargewicht der Polyoxyalkylen- oder Polyalkyleniminreste liegt vorzugsweise in einem Bereich von etwa 400 bis 50000, besonders bevorzugt 800 bis 20000, speziell 2000 bis 10000.

Weist ein Substituent mehrere Reste R auf, so können diese gleiche oder verschiedene Bedeutungen aufweisen.

Bei den Strukturen der Formeln I und II kann es sich um Phosphacyclohexanone handeln, sofern zwei geminale Reste, ausgewählt R1 bis R¹⁰, für =O stehen. Vorzugsweise stehen in diesem Fall R⁵ und R⁶ in Formel I für ein doppelt gebundenes Sauerstoffatom.

Vorzugsweise sind in den Phosphacyclohexanen der Formeln I und II mindestens zwei oder drei oder vier der Reste R1 bis R¹⁰ von Wasserstoff verschieden. Vorzugsweise enthalten mindestens einer, zwei oder drei der Reste R und R¹ bis R¹⁰ cyclische Strukturen, die aliphatisch, aromatisch oder heterocyclisch sein können. In den Verbindungen der Formel I liegen die cyclischen Strukturen beispielsweise in den Positionen 2, 4 und 6 vor. Die Strukturen können auch beispielsweise in den Positionen 1, 2 und 6 vorliegen.

Bevorzugt stehen die Reste R1 bis R¹⁰ für Wasserstoff und Reste, wie sie für R definiert sind, insbesondere C₁₋₁₂-Alkylreste, C₇₋₁₃-Aralkylreste, C₇₋₁₃-Alkarylreste und/oder C₆₋₁₂-Arylreste. Die Alkylreste können cyclische Strukturen enthalten. Die Arylgruppen der Aralkylreste, Alkarylreste und Arylreste leiten sich vorzugsweise von Benzol oder Naphthalin ab. Beispielsweise kann es sich um Phenylreste (R¹ bis R¹⁰) oder Naphthylreste handeln. Alkarylreste weisen dabei vorzugsweise einen, zwei oder drei Alkylsubstituenten auf, bei denen es sich insbesondere um Methyl- oder Ethylreste handelt.

Wenn R und/oder R1 bis R¹⁰ für Alkyl- und Arylreste stehen, so können diese fluoriert oder perfluoriert sein. Ein bevorzugter fluorierter Alkylrest ist Trifluormethyl.

In einer geeigneten Ausführungsform der Erfindung steht in den Verbindungen der allgemeinen Formel I oder II wenigstens einer der Reste R oder R¹ bis R¹⁰ für eine polare (hydrophile) Gruppe, wobei dann in der Regel wasserlösliche Katalysatoren resultieren. Bevorzugt sind die polaren Gruppen ausgewählt unter W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR' oder W'((CH₂)₄O)ₓR', worin W', M⁺, X-, x und R' die zuvor angegebenen Bedeutungen besitzen.

Mindestens einer der Substituenten R und R1 bis R¹⁰ kann eine zusätzliche, zur Koordination befähigte dreibindige Phosphor- oder Stickstoffgruppierung aufweisen, wodurch ein zwei- oder mehrzähniger Ligand entsteht. Besonders bevorzugt sind Phosphan-, Phosphinit-, Phosphonit- und Phosphitgruppen sowie η⁵-Phospholylkomplexe oder Phosphabenzolgruppierungen.

Vorzugsweise handelt es sich bei den Resten R1 bis R¹⁰ um Kohlenwasserstoffreste, die keine weiteren Heteroatome aufweisen.

Bei den Brücken W und W' handelt es sich nach einer bevorzugten Ausführungsform um Einfachbindungen oder Brücken mit 1 bis 6 Kohlenstoffatomen, die Bestandteil einer cyclischen oder aromatischen Gruppe sein können. Es kann sich dabei um Einfachbindungen handeln, wie auch um niedere Alkylengruppen, wie z. B. C₁-₁₀-Alkylen.

In den Verbindungen der Formel II können die beiden Reste R gemeinsam und/oder eine Gruppe W für eine Brücke mit 1 bis 20 Kohlenstoffatomen stehen, die Bestandteil einer cyclischen oder aromatischen Gruppe sein kann und/oder die durch Heteroatome unterbrochen sein kann. Nach einer ersten bevorzugten Ausführungsform stehen die verbrückenden Gruppen für eine C₁₋₂₀-Alkylenkette. Verbrückende Alkylenketten sind gegebenenfalls mit Cycloalkyl, Heterocycloalkyl, Aryl und/oder Hetaryl substituiert, welche gegebenenfalls 1, 2 oder 3 der zuvor genannten Substituenten tragen können. Verbrückende Alkylenketten können in Abhängigkeit von der Kohlenstoffatomanzahl der Alkylenkette 1, 2, 3 oder 4 Doppelbindungen aufweisen und/oder durch 1 bis 10, z. B. 1, 2 oder 3, nicht benachbarte, gegebenenfalls substituierte Heteroatome unterbrochen sein und/oder ein-, zwei- oder dreifach mit Cycloalkyl, Aryl oder Hetaryl anelliert sein. Bevorzugt handelt es sich um C₁₋₁₅-, besonders bevorzugt um C₁₋₁₀-Alkylenketten, wie beispielsweise C₆- oder C₃-Alkylenketten.

Wenn in den Verbindungen der Formel II die beiden Reste R gemeinsam und/oder eine Gruppe W für eine Aryl-anellierte Alkylenbrücke stehen, so handelt es sich bei den anellierten Arylen bevorzugt um Benzol oder Naphthalin.

Anellierte Benzolringe sind vorzugsweise unsubstituiert oder weisen einen, zwei oder drei, insbesondere einen oder zwei Substituenten auf, die ausgewählt sind unter Alkyl, Alkoxy, Halogen, SO₃H, Sulfonat, NE¹E², Alkylen-NE¹E², Trifluormethyl, Nitro, Carboxyl, Alkoxycarbonyl, Acyl und Cyano. Anellierte Naphthaline sind vorzugsweise unsubstituiert oder weisen im nicht anellierten Ring und/oder im anellierten Ring jeweils einen, zwei oder drei, insbesondere einen oder zwei der zuvor bei den anellierten Benzolringen genannten Substituenten auf.

Vorzugsweise handelt es sich bei den verbrückenden Gruppen um eine unsubstituierte C₂₋₆-Alkylenbrücke.

Bevorzugt stehen die beiden Reste R gemeinsam und/oder eine Gruppe W weiterhin für eine C₂₋₂₀-Alkylenbrücke, die durch bis zu 20, insbesondere durch bis zu 10, nicht benachbarte, gegebenenfalls substituierte Heteroatome unterbrochen ist. Diese sind vorzugsweise ausgewählt unter O, S, NR^{α} oder SiR^{β}R^{γ}, wobei die Reste R^{α}, R^{β} oder R^{γ} unabhängig voneinander für Alkyl, Cycloalkyl oder Aryl stehen. Vorzugsweise handelt es sich um oligomere Polyoxyalkylen- oder Polyalkyleniminbrücken. Diese weisen beispielsweise die zuvor beschriebenen Wiederholungseinheiten auf.

Nach einer weiteren Ausführungsform können in den Verbindungen der. Formel II die beiden Reste R gemeinsam und/oder eine Gruppe W auch eine höhermolekulare Polyoxyalkylen- oder Polyalkylenimin-Brücke mit mindestens 21 Kohlenstoffatomen bilden. Das zahlenmittlere Molekulargewicht der Polyoxyalkylen- oder Polyalkyleniminreste liegt vorzugsweise in einem Bereich von etwa 400 bis 50000, besonders bevorzugt 800 bis 20000 und speziell 1000 bis 10000. Besonders bevorzugt handelt es sich um Polyethylenoxide, Copolymere aus Ethylenoxid und 1,2-Propylenoxid, in denen die Alkylenoxide in beliebiger Reihenfolge, alternierend oder in Form von Blöcken eingebaut sein können sowie Polyethylenimine.

Nach einer geeigneten Ausführungsform kann in den Verbindungen der Formel I auch einer der Reste R oder R¹ bis R¹⁰ und in den Verbindungen der Formel II auch einer der Reste R oder R¹ bis R⁸ oder die beiden Reste R gemeinsam oder eine Gruppe W auch für einen von den zuvor genannten Definitionen für diese Reste und Gruppen verschiedenen Polymerrest mit einem zahlenmittleren Molekulargewicht im Bereich von etwa 500 bis 50000 stehen. Die Wiederholungseinheiten dieser Polymerreste leiten sich formal ab von Monomeren, die ausgewählt sind unter Mono- und Diolefinen, Vinylaromaten, Estern α,β-ethylenisch ungesättigter Mono- und Dicarbonsäuren mit C₁-C₃₀-Alkanolen, N-Vinylamiden, N-Vinyllactamen, unter Ringöffnung polymerisierbaren heterocyclischen Verbindungen und Mischungen davon.

Bevorzugt weisen die Polymerreste ein zahlenmittleres Molekulargewicht im Bereich von 800 bis 20000, besonders bevorzugt 2000 bis 10000, auf.

Als Monomere bevorzugte Monoolefine sind C₂₋₈-Monoolefine, wie Ethen, Propen, n-Buten, Isobuten sowie Aromaten-substituierte Monoolefine, wie 1,1-Diphenylethylen, 1,2-Diphenylethylen und Mischungen der zuvor genannten Monoolefine. Als Monomere bevorzugte Diolefine sind konjugierte Diene, wie Butadien, Isopren, 2,3-Dimethylbutadien, Piperylen(1,3-Pentadien) und Mischungen davon. Die Ester α,β-ethylenisch ungesättigter Mono- und Dicarbonsäuren sind vorzugsweise ausgewählt unter den Estern der Acrylsäure, Methacrylsäure, Maleinsäure, Fumarsäure, Itaconsäure und Crotonsäure. Bevorzugt sind die Ester mit C₁₋₂₀-Alkanolen. Dazu zählen beispielsweise Methyl(meth)acrylat, Ethyl(meth)acrylat, n-Butyl(meth)acrylat, tert.-Butyl(meth)acrylat, n-Hexyl(meth)acrylat, n-Octyl(meth)acrylat, Ethylhexyl(meth)acrylat, etc. Als Monomere geeignete Vinylaromaten sind beispielsweise Styrol, α-Methylstyrol, o-Chlorstyrol, Vinyltoluole und Mischungen davon. Geeignete n-Vinylamide sind beispielsweise n-Vinylformamid, n-Vinylacetamid, n-Vinylpropionamid und Mischungen davon. Geeignete n-Vinyllactame sind beispielsweise n-Vinylpyrrolidin, n-Vinylpiperidon, n-Vinylcaprolactam und Mischungen davon. Geeignete Monomere für die Ringöffnungspolymerisation sind z. B. cyclische Ether, wie Ethylenoxid und Propylenoxid, cyclische Amine, cyclische Sulfide (Ethylensulfid, Thietane), Lactone und Lactame. Bevorzugt sind ε-Caprolacton und ε-Caprolactam.

Die zuvor genannten Monomere können einzeln, in Form von Gemischen aus der jeweiligen Monomerklasse sowie allgemein als Mischungen eingesetzt werden.

Die Herstellung der als Reste geeigneten Polymere erfolgt nach üblichen, dem Fachmann bekannten Polymerisationsverfahren. In Abhängigkeit von den zu polymerisierenden Monomeren zählt dazu die radikalische, kationische und anionische Polymerisation, einschließlich der kationischen und anionischen Ringöffnungspolymerisation.

Erfolgt die Herstellung der Polymerreste durch anionische Polymerisation, beispielsweise durch die entsprechende im Folgenden beschriebene Reaktionsvariante zur Herstellung der erfindungsgemäßen Phosphacyclohexane, so werden als Monomere vorzugsweise Akzeptor-aktivierte ethylenisch ungesättigte Verbindungen und_Ethen eingesetzt.

Wenn in den Verbindungen der Formel I einer der Reste R oder R¹ bis R¹⁰ und in den Verbindungen der Formel II einer der Reste R oder R¹ bis R⁸ oder die beiden Reste R gemeinsam oder eine Gruppe W für einen Polymerrest stehen, so handelt es sich vorzugsweise um einen Polyolefinrest (Polyalkenrest). Diese Polyolefine weisen Wiederholungseinheiten auf, die sich von einpolymerisierten Monomeren ableiten, die vorzugsweise ausgewählt sind unter C₂₋₆-Alkenen, wie Ethen, Propen, n-Buten, Isobuten, Olefinen mit zwei Doppelbindungen, wie Butadien, Isopren, 1,3-Pentadien und Mischungen davon. Polyolefine, die konjugierte Diene eingebaut enthalten, können im Wesentlichen ausschließlich die 1,2- und 1,4-Polymerisationsprodukte als auch Mischformen mit beliebigen 1,2- und 1,4-Anteilen aufweisen. Verfahren zur Einstellung der 1,2- und 1,4-Anteile bei der Polymerisation konjugierte Diene sind dem Fachmann bekannt. Dazu zählt beispielsweise bei der anionischen Polymerisation der Zusatz von Donorlösungsmitteln, z. B. Ether, wie THF, oder Aminen. Polyolefine mit Wiederholungseinheiten von 1,2-Additionsprodukten konjugierter Diene weisen seitenständige ethylenisch ungesättigte Gruppen auf. Polyolefine mit Wiederholungseinheiten von 1,4-Additionsprodukten weisen in der Hauptkette ethylenisch ungesättigte Gruppen auf. Diese können gewünschtenfalls teilweise oder vollständig hydriert werden. Möglich ist jedoch auch die Verwendung von Phosphacyclohexanen mit Polyolefinresten mit ethylenisch ungesättigten Seitenketten als Liganden in Übergangsmetallkomplexen für die Hydroformylierung. Dabei erfolgt unter Hydroformylierungsbedingungen in der Regel zumindest eine teilweise Umsetzung der ethylenisch ungesättigten Seitenketten in Alkoholgruppen, d. h. es resultieren Liganden mit polaren Seitenketten.

Wenn in den Verbindungen der Formel I einer der Reste R oder R¹ bis R¹⁰ und in den Verbindungen der Formel II einer der Reste R oder R¹ bis R⁸ oder die beiden Reste R gemeinsam oder eine Gruppe W für einen Polyolefinrest stehen, so handelt es sich vorzugsweise um einen Polyethylen- oder Polybutadienrest.

Die in Struktur II nicht an die Brücke W gebundenen Positionen der Phosphorcyclohexanringe können zudem einen der Reste R bzw. R1 bis R¹⁰ tragen.

Der Rest R' ist vorzugsweise Wasserstoff oder ein C₁₋₆-Alkyl, wie ein Methyl- oder Ethylrest. Weist ein Substituent mehrere Reste R' auf, so können diese gleiche oder verschiedene Bedeutungen aufweisen.

Vorzugsweise liegt, abgesehen von C=O in 4-Position und C(CH₃)₂ in 2- und/oder 6-Position an jedem Ringkohlenstoffatom des Phosphacyclohexans maximal ein von Wasserstoff verschiedener Substituent vor. Beispielsweise können in 2- und 6-Position, 2-, 4- und 6-Position, 2-, 3-, 5- und 6-Position Substituenten vorliegen. Besonders bevorzugt sind Substituenten, speziell Aryl, in 2- und 6-Position.

Bevorzugt werden als Liganden Phosphacyclohexane verwendet, die ausgewählt sind unter Verbindungen der allgemeinen Formel III 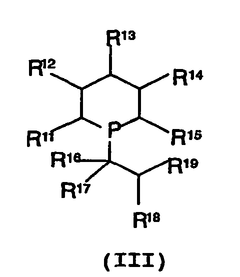 mit der Bedeutung:
- R¹¹ bis R¹⁹: unabhängig voneinander Wasserstoff, C₁₋₂₀-Alkyl, C₇₋₂₀-Aralkyl, C₇₋₂₀-Alkaryl, C₆₋₁₂-Aryl, wobei eines oder mehrere Kohlenstoffatome durch Beteroatome ersetzt sein können, W'COO⁻M⁺, W'SO₃⁻M⁺, W'PO₃²⁻M⁺₂, W'NR'₃⁺X⁻, W'OR', WNR'₂, W'COOR', W'SR', W'(CHR'CH₂O)ₓR', W'(CH₂NR')ₓR', W'(CH₂CH₂NR')ₓR',
wobei jeweils zwei vicinale Reste R¹¹ bis R¹⁵ und/oder R¹⁷ und R¹⁸ und/oder R¹⁶ und R¹⁷ und/oder R¹⁶ und R¹⁹ und/oder R¹⁸ und R¹⁹ zu Ringen verbunden sein können,
- W': Einfachbindung oder Brücke mit 1 bis 20 Kohlenstoffatomen, die Bestandteil einer cyclischen oder aromatischen Gruppe sein kann,
- R': Wasserstoff oder C1-6-Alkyl,
- M⁺: Kation,
- X⁻: Anion,
- x: Zahl von 1 bis 240,
wobei einer oder mehrere der Reste R¹¹ bis R¹⁹ eine zusätzliche, zur Koordination befähigte dreibindige Phosphor- oder Stickstoffgruppierung aufweisen können,
wobei R¹⁸ auch -W'-CR^{20'}=CR^{21'}R²², wobei R²⁰, R²¹, R²² wie vorstehend für R¹¹ bis R¹⁹ definiert sind, bedeuten kann

Besonders bevorzugt werden als Liganden Phosphacyclohexane verwendet, die ausgewählt sind unter Verbindungen der allgemeinen Formeln I.a bis I.g und II.a 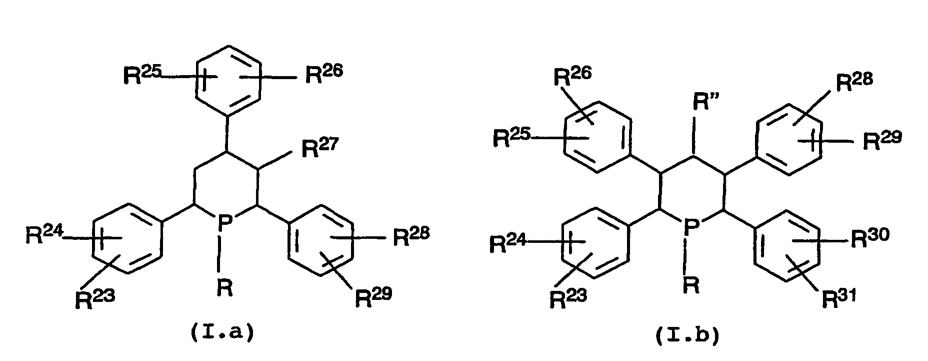 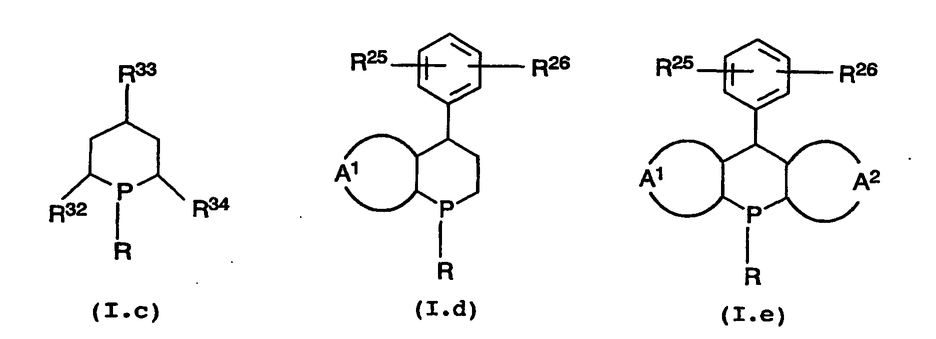 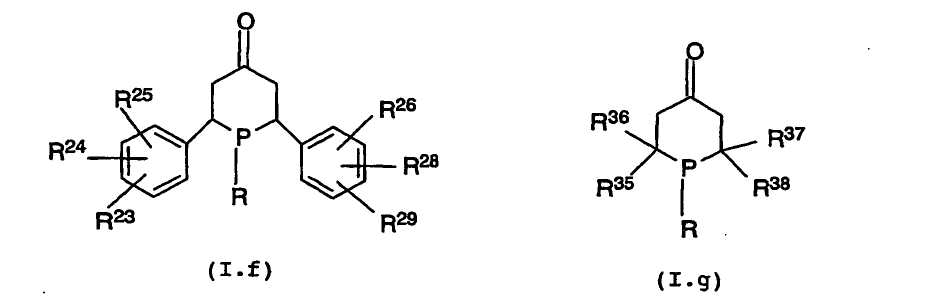 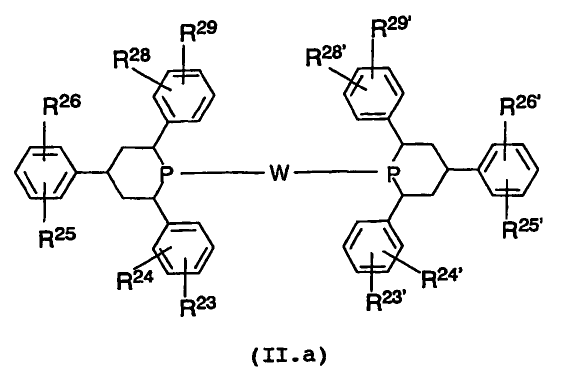 worin
- R: für C₁₋₂₀-Alkyl, Cycloalkyl, C₆₋₁₂-Aryl, W'(CHR'CH₂O)ₓR', W'((CH₂)₄O)ₓR' oder einen Polymerrest mit einem zahlenmittleren Molekulargewicht im Bereich von 500 bis 50000, der aufgebaut ist aus Ethylen und/oder Butadien, steht, wobei
W' für eine Einfachbindung oder C₁₋₄-Alkylen steht, und
R' für Wasserstoff oder C₁₋₂₀-Alkyl steht,
x für eine ganze Zahl von 1 bis 240 steht,
- R²³, R^{23'}, R²⁴, R^{24'}, R²⁵, R^{25'}, R²⁶, R^{26'}, R²⁸, R^{28'}, R²⁹, R^{29'}, R³⁰ und R³¹: unabhängig voneinander für Wasserstoff, Alkyl, Alkoxy, Carboxyl, Carboxylat, Trifluormethyl, -SO₃H, Sulfonat, NE¹E² oder Alkylen-NE¹E² stehen, wobei E¹ und E² unabhängig voneinander für Wasserstoff, Alkyl oder Cycloalkyl stehen,
- R²⁷: für Wasserstoff, Alkyl, Cycloalkyl, Aryl oder Aralkyl steht,
- R³², R³³, R³⁴, R³⁵, R³⁶, R³⁷ und R³⁸: unabhängig voneinander für Alkyl oder Cycloalkyl stehen,
- R'': für Wasserstoff oder Phenyl steht,
- A¹und A²: zusammen mit den benachbarten Kohlenstoffatomen des Phosphacyclohexans, an die sie gebunden wird, für ein anelliertes Ringsystem mit je 1 oder 2 weiteren Ringen stehen,
- W: für eine Brücke mit 1 bis 20 Kohlenstoffatomen steht, die durch Heteroatome unterbrochen sein kann.

In den Verbindungen der Formel I.a bis I.f und II.a stehen die Reste R²³, R^{23'}, R²⁴, R^{24'}, R²⁵, R^{25'}, R²⁶, R^{26'}, R²⁸, R^{28'}, R²⁹, R^{29'}, R³⁰ und R³¹ vorzugsweise unabhängig voneinander für Wasserstoff, C₁₋₄-Alkyl, bevorzugt Methyl, Ethyl, Isopropyl, tert.-Butyl, C₁₋₄-Alkoxy, bevorzugt Methoxy.

R²⁷ steht vorzugsweise für Aralkyl, insbesondere Benzyl.

R³², R³³ und R³⁴ stehen vorzugsweise unabhängig voneinander für C₁₋₄-Alkyl, besonders bevorzugt tert.-Butyl.

R³⁵, R³⁶, R³⁷ und R³⁸ stehen vorzugsweise unabhängig voneinander für C₁₋₄-Alkyl, besonders bevorzugt Methyl.

Bei den Ringen der anellierten Ringsystemen A¹ und A² handelt es sich vorzugsweise um 5- bis 7-gliedrige Ringe. Bevorzugt sind Ringsysteme, die sich von Benzol, Naphthalin und deren teilweisen Hydrierungsprodukten oder Perhydrierungsprodukten ableiten. Ankondensierte Ringe sind vorzugsweise unsubstituiert oder weisen je Ring 1,2 oder 3, insbesondere 1 oder 2 Substituenten auf, die ausgewählt sind unter Alkyl, Alkoxy, Halogen, SO₃H, Sulfonat, NE¹E², Alkylen-NE¹E², Trifluormethyl, Nitro, Carboxyl, Alkoxycarbonyl, Acyl und Cyano.

In der Formel II.a steht der Rest W vorzugsweise für eine C₁₋₁₀-Alkylengruppe, wie beispielsweise eine C₂-C₈-Alkylengruppe. Die Gruppe W steht vorzugsweise weiterhin für eine Brücke mit 1 bis 20 Kohlenstoffatomen, die durch bis zu 10 nicht benachbarte Sauerstoffatome unterbrochen sein kann. Dabei handelt es sich dann um niedermolekulare Polyoxyalkylengruppen, die Wiederholungseinheiten aufweisen, die sich von Ethylenoxid, Propylenoxid, Tetrahydrofuran und beliebigen Kombinationen davon ableiten. Die Gruppe W kann weiterhin für eine Polyoxyalkylengruppe mit mehr als 21 Kohlenstoffatomen stehen.

Bevorzugt sind Verbindungen der Formel I.a, worin R²³, R²⁴, R²⁵, R²⁶, R²⁷, R²⁸, und R²⁹ für Wasserstoff stehen. Bevorzugt sind weiterhin Verbindungen der Formel I.a, worin R²⁵, R²⁶ und R²⁷ für Wasserstoff und R²³, R²⁴, R²⁸ und R²⁹ für C₁₋₄-Alkyl, besonders bevorzugt Methyl, stehen. Bevorzugt sind weiterhin Verbindungen der Formel I.a, worin R²³ und R²⁸ für C₁₋₄-Alkyl, besonders bevorzugt Methyl, und R²⁴, R²⁵, R²⁶, R²⁷ und R²⁹ für Wasserstoff stehen. Vorzugsweise weisen die Phenylreste in 2- und 6-Position des Phosphacyclohexanrings der Verbindungen der Formel I.a je einen Alkylrest in der 2-Position oder zwei Alkylreste in 2- und 4-Position auf. Vorzugsweise steht der Rest R in den Verbindungen der Formel I.a (wie auch I.b bis I.f und II.a) für C₁₋₁₄-Alkyl, wie Propyl, n-Butyl, sek.-Butyl, Isobutyl, n-Octyl, 2-Ethylhexyl, Dodecyl, sowie Cyclohexyl, Methoxyethoxyethyl oder für einen Polymerrest, beispielsweise einen Polyethylen- oder Polybutadienrest.

Bevorzugt sind in den Verbindungen der Formel I.b die Reste R²³, R²⁴, R²⁵, R²⁶, R²⁸, R²⁹, R³⁰ und R³¹ ausgewählt unter Wasserstoff und C₁₋₄-Alkyl, besonders bevorzugt Wasserstoff.

Besonders bevorzugt stehen in den Verbindungen der Formel I.c die Reste R³², R³³ und R³⁴ für tert.-Butyl.

Besonders bevorzugt steht die Verbindung der Formel I.e für ein 1,2,7,8-Dibenzo-3,4,5,6-tetrahydro-9-phosphaanthracengerüst.

Besonders bevorzugt sind in den Verbindungen der Formel I.f die Reste R²³, R²⁴, R²⁵, R²⁶, R²⁸ und R²⁹ ausgewählt unter Wasserstoff und C₁₋₄-Alkyl, besonders bevorzugt Methyl. Vorzugsweise weisen die Phenylreste der Phosphacyclohexanone der Formel I.f einen von Wasserstoff verschiedenen Substituenten in der 2-Position, zwei von Wasserstoff verschiedene Substituenten in der 2- und 4-Position oder drei von Wasserstoff verschiedene Substituenten in der 2-, 4- und 6-Position auf.

Besonders bevorzugt sind Verbindungen der Formel I.g, worin R³⁵, R³⁶, R³⁷ und R³⁸ für C₁₋₄-Alkyl, insbesondere Methyl, stehen.

Bezüglich bevorzugter Substituenten der Verbindungen der Formel II.a gilt das zuvor für Verbindungen der Formel I.a Gesagte. Die verbrückende Gruppe W steht vorzugsweise für eine C₁₋₁₀-Alkylengruppe, wie beispielsweise eine C₂-C₈-Alkylengruppe.

Für das erfindungsgemäße Verfahren geeignete Katalysatorsysteme können andere Komplexbildner als die oben erwähnten phosphorhaltigen Liganden aufweisen. Beispiele dafür sind etwa Carbonyl- oder Hydridgruppen, stickstoffhaltige Liganden, beispielsweise Amine, Amide oder Stickstoffheterocyclen, Sulfate, Nitrate oder Carboxylate. Rhodium-Katalysatoren ohne Phosphinliganden, die für die Verwendung in dem erfindungsgemäßen Verfahren geeignet sind, werden nachfolgend auch als "nackte" Rhodium-Katalysatoren bezeichnet.

Beispiele für "nackte" Rhodium-Katalysatoren und deren Verwendung bei der Hydroformylierung von Olefinen finden sich etwa in Chem. Ber. 102, 2238 (1969), Tetrahedron Lett. 29, 3261 (1968) und Hydrocarbon Process. 85 - 86 (1975). Auch Patentanmeldungen bzw. Patente existieren zu diesem Gebiet, so die US 4,400,547, DE-A-33 38 340, DE-A-26 04 545 und WO 82/03856.

Besonders geeignet für die Verwendung gemäß der vorliegenden Erfindung sind weiterhin die in der DE-A-198 01 437 beschriebenen Rhodium-Katalysatoren. Dabei handelt es sich um Rhodium-Katalysatoren, die als Liganden derivatisierte, im Wesentlichen nicht wasserlösliche und zur Komplexbildung befähigte Polyamine mit einem mittleren Molekulargewicht > 1000 Dalton mit mindestens 10 Stickstoffatomen tragen.

Insbesondere handelt es sich bei den Liganden um derivatisierte Polyamine mit einem mittleren Molekulargewicht von mehr als 1000 Dalton. In der meist bevorzugten Ausführungsform handelt es sich bei dem Polyamin um ein Polyethylenamin, das im Wesentlichen aus Einheiten der nachstehenden Formel (IV) besteht.

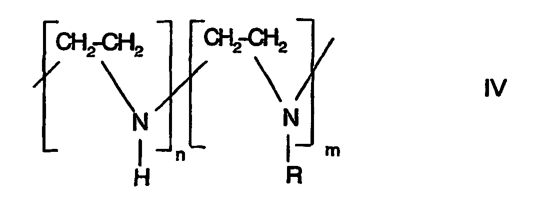

In der Formel I ist die Summe aus m + n mindestens 10 und das Verhältnis aus m/m + n = 0,01 bis 1; R bezeichnet gleiche oder verschiedene Alkyl-, Cycloalkyl-, Aryl-, Aralkyl-, Alkylcarbonylreste mit bis zu 30 C-Atomen oder Hydroxyalkyl(poly)oxyalkylenreste mit bis zu 500 Alkylenoxy-Einheiten.

Die in der DE-A-198 01 437 offenbarten Rhodiumkomplexe sind ein integraler Bestandteil der vorliegenden Erfindung und durch Referenz in die vorliegende Anmeldung einbezogen.

Die Einsatzmenge des erfindungsgemäß eingesetzten Rhodium-Katalysators liegt im Allgemeinen im Bereich von etwa 1 bis 150 ppm, bevorzugt bei 1 bis 100 ppm. Die Reaktionstemperatur liegt im Allgemeinen in einem Bereich von Raumtemperatur bis 200 °C, bevorzugt 50 bis 180 °C, insbesondere 80 bis 150 °C. Die Reaktion kann bei einem erhöhten Druck von etwa 10 bis 1000 bar, bevorzugt 20 bis 650 bar, insbesondere 80 bis 350 bar, durchgeführt werden.

Das Molmengenverhältnis von H₂:CO beträgt im Allgemeinen etwa 1:5 bis etwa 5:1, bevorzugt 1:2 bis 2:1.

Die bei der Hydroformylierung resultierenden Aldehyde bzw. Aldehyd/Alkohol-Gemische können vor der Hydrierung gewünschtenfalls nach üblichen, dem Fachmann bekannten Verfahren isoliert und gegebenenfalls gereinigt werden. Vorzugsweise wird vor der Hydrierung der Hydroformylierungskatalysator aus dem Reaktionsgemisch entfernt. Er kann im Allgemeinen, gegebenenfalls nach Aufarbeitung, erneut zur Hydroformylierung eingesetzt werden. Zur Hydrierung werden die bei der Hydroformylierung erhaltenen Reaktionsgemische mit Wasserstoff in Gegenwart eines Hydrierungskatalysators umsetzt.

Geeignete Hydrierungskatalysatoren sind im Allgemeinen Übergangsmetalle, wie z. B. Cu, Cr, Mo, W, Fe, Rh, Co, Ni, Pd, Pt, Ru, etc., oder deren Mischungen, die zur Erhöhung der Aktivität und Stabilität auf Trägern, wie z. B. Aktivkohle, Aluminiumoxid, Siliciumdioxid, Kieselgur, etc., aufgebracht werden können. Zur Erhöhung der katalytischen Aktivität können Fe, Co, und bevorzugt Ni auch in Form der Raney-Katalysatoren als Metallschwamm mit einer sehr großen Oberfläche verwendet werden. Bevorzugt werden technisch etablierte Heterogenkatalysatoren eingesetzt, wie Aktivmassen auf Trägern oder Vollkontakte, die z. B. in Riesel- oder Sumpffahrweise oder in Suspension eingesetzt werden. Bevorzugt liegt der Druck in einem Bereich von etwa 5 bis 350 bar. Vorzugsweise wird Wasserstoff in geringem molaren Überschuss eingesetzt.

Auch andere Verfahren können zur Reduktion der Aldehyde zu den Alkoholen verwendet werden. Dazu zählen z. B. die Reduktion mit komplexen Hydriden, wie z. B. LiAlH₄ und NaBH₄, die Reduktion mit Natrium in Ethanol nach Bouveault-Blanc sowie weitere bekannte Verfahren.

Die erfindungsgemäßen Alkoholgemische sind nach dem beschriebenen erfindungsgemäßen Verfahren in vorteilhafter Weise erhältlich. Wird ein großtechnisch zugängiges C₁₂-C₁₄-Monoolefingemisch nach dem erfindungsgemäßen Verfahren in Gegenwart eines Rhodium-Katalysators hydroformyliert und nachfolgend hydriert, so wird ein C₁₃-C₁₅-Alkoholgemisch erhalten, das eine andere Isomerenverteilung aufweist, als etwa ein C₁₃-C₁₅-Alkoholgemisch, das durch Kobalt-katalysierte Hydroformylierung hergestellt wurde. Dabei führen die erfindungsgemäßen Alkoholgemische nach der Verarbeitung zu oberflächenaktiven Verbindungen mit vorteilhaften Eigenschaften. Dazu zählen z. B. eine gute biologische Abbaubarkeit, ein geringes Schaumvermögen und ein sich über einen kleinen Konzentrationsbereich erstreckender Gelbereich.

Ein weiterer Gegenstand der Erfindung ist ein Verfahren zur Herstellung funktionalisierter Alkoholgemische, wobei man ein Alkoholgemisch, wie zuvor beschrieben, einer Alkoxilierung, Glycosidierung, Sulfatierung, Phosphatierung, Alkoxilierung und nachfolgender Sulfatierung oder Alkoxilierung und nachfolgender Phosphatierung unterwirft.

Gegenstand der Erfindung ist somit ein Verfahren zur Herstellung von funktionalisierten Alkoholgemischen, wobei man
a) ein Monoolefingemisch bereitstellt, das im Wesentlichen Olefine mit 12 und 14 Kohlenstoffatomen enthält und das lineare α-Olefine und einen Anteil von 5 bis 20 Gew.-% an davon verschiedenen Olefinen aufweist,
b) das Monoolefingemisch durch Umsetzung mit Kohlenmonoxid und Wasserstoff in Gegenwart eines Rhodiumkatalysators hydroformyliert und nachfolgend hydriert,
c) das Alkoholgemisch aus Schritt b) einer Alkoxilierung, Glycosidierung, Sulfatierung, Phosphatierung, Alkoxilierung und nachfolgender Sulfatierung oder Alkoxilierung und nachfolgender Phosphatierung, unterwirft.

Die Alkoxilierung der Alkoholgemische erfolgt durch Umsetzung mit mindestens einem Alkylenoxid. Bevorzugt sind die Alkylenoxide ausgewählt unter Verbindungen der allgemeinen Formel II 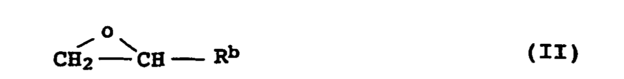 worin
R^{b} für Wasserstoff oder einen geradkettigen oder verzweigten C₁- bis C₁₆-Alkylrest steht,
und Mischungen davon.

Bevorzugt steht der Rest R^{b} in der Formel II für einen geradkettigen oder verzweigten C₁- bis C₈-Alkylrest, insbesondere C₁- bis C₄-Alkylrest.

Bevorzugt sind die Alkylenoxide ausgewählt unter Ethylenoxid, Propylenoxid, Butylenoxid, Pentylenoxid und Mischungen davon.

Die Umsetzung der Alkoholgemische mit dem/den Alkylenoxid(en) erfolgt nach üblichen, dem Fachmann bekannten Verfahren und in dafür üblichen Apparaturen.

Die mittlere Kettenlänge der Polyetherketten der so funktionalisierten Alkoholgemische kann durch das Molmengenverhältnis von Alkohol zu Alkylenoxid bestimmt werden. Bevorzugt werden alkoxilierte Alkoholgemische mit etwa 1 bis 200, bevorzugt etwa 1 bis 100, insbesondere 1 bis 50 Alkylenoxideinheiten hergestellt.

Die Alkoholgemische können gewünschtenfalls nur mit einem Alkylenoxid oder mit zwei oder mehreren verschiedenen Alkylenoxiden umgesetzt werden. Bei der Umsetzung der Alkoholgemische mit einem Gemisch aus zwei oder mehreren Alkylenoxiden enthalten die resultierenden Alkoxilate die Alkylenoxideinheiten im Wesentlichen statistisch verteilt. Werden die Alkylenoxide getrennt nacheinander eingesetzt, so resultieren Alkoxilate, die entsprechen der Zugabereihenfolge die Alkylenoxideinheiten in Form von Blöcken einpolymerisiert enthalten.

Die Alkoxilierung kann durch starke Basen, wie Alkalihydroxide und Erdalkalihydroxide, Brönstedsäuren oder Lewissäuren, wie AlCl₃, BF₃ etc. katalysiert werden.

Die Alkoxilierung erfolgt vorzugsweise bei Temperaturen im Bereich von etwa 80 bis 250 °C, bevorzugt etwa 100 bis 220 °C. Der Druck liegt vorzugsweise zwischen Umgebungsdruck und 600 bar. Gewünschtenfalls kann das Alkylenoxid eine Inertgasbeimischung, z.B von etwa 5 bis 60 %, enthalten.

Die durch Alkoxilierung erhaltenen funktionalisierten Alkoholgemische zeigen eine sehr gute Oberflächenaktivität und können als nichtionische Tenside in einer Vielzahl von Anwendungsbereichen vorteilhaft eingesetzt werden.

Die erfindungsgemäß hergestellten Alkoholalkoxilate können gewünschtenfalls durch Umsetzung mit Alkylierungsreagenzien, beispielsweise Methylchlorid, Dimethylsulfat, Diethylsulfat, Butylchlorid und Isobuten zu endgruppen-alkylierten Tensiden umgesetzt werden.

Die Glycosidierung der Alkoholgemische erfolgt durch ein-, zwei- oder mehrfache Umsetzung der erfindungsgemäßen Alkoholgemische mit Mono-, Di- oder Polysacchariden. Die Umsetzung erfolgt nach üblichen, dem Fachmann bekannten Verfahren. Dazu zählt zum einen die säurekatalysierte Umsetzung unter Wasserentzug. Geeignete Säuren sind z. B. Mineralsäuren, wie HCl und H₂SO₄. Dabei werden in der Regel Oligosaccharide mit statistischer Kettenlängenverteilung erhalten. Vorzugsweise liegt der durchschnittliche Oligomerisierungsgrad bei 1 bis 3 Saccharidresten. Nach einem weiteren geeigneten Verfahren kann das Saccharid zunächst durch Umsetzung mit einem niedermolekularen C₁- bis C₈-Alkanol, wie z. B. Ethanol, Propanol oder Butanol, acetalisiert werden. Die Acetalisierung erfolgt vorzugsweise säurekatalysiert. Das dabei resultierende Glycosid mit dem niedermolekularen Alkohol kann anschließend mit einem erfindungsgemäßen Alkoholgemisch zu den entsprechenden Glycosiden umgesetzt werden. Für diese Reaktion eignen sich im Allgemeinen auch wässrige Saccharidlösungen. Nach einem weiteren geeigneten Verfahren kann das Saccharid zunächst durch Umsetzung mit einem Halogenwasserstoff in das entsprechende O-Acetylhalosaccharid überführt und anschließend mit einem erfindungsgemäßen Alkoholgemisch in Gegenwart säurebindender Verbindungen glycosidiert werden.

Vorzugsweise werden zur Glycosidierung Monosaccharide eingesetzt. Insbesondere werden Hexosen, wie Glucose, Fructose, Galactose, Mannose etc. und Pentosen, wie Arabinose, Xylose, Ribose etc. eingesetzt. Besonders bevorzugt wird Glucose eingesetzt. Die Saccharide können einzeln oder in Form von Gemischen eingesetzt werden. Bei Saccharidgemischen resultieren im Allgemeinen Glycoside mit statistisch verteilten Zuckerresten. Bei mehrfacher Saccharidanlagerung an eine alkoholische Hydroxidgruppe resultieren Polyglycoside der erfindungsgemäßen Alkoholgemische. Auch zu Polyglycosidierung können mehrere Saccharide nacheinander oder als Gemisch eingesetzt werden, so dass die resultierenden funktionalisierten Alkoholgemische die Saccharide in Form von Blöcken oder statistisch verteilt eingebaut enthalten. Es können je nach Reaktionsbedindungen, insbesondere Reaktionstemperatur, Furanose-oder Pyranosestrukturen resultieren.

Geeignete Verfahren und Reaktionsbedingungen zur Glycosidierung sind z. B. in Ullmanns Encyclopedia of Industrial Chemistry, 5. Aufl., Bd. A25 (1994), S. 792-793 und den dort zitierten Dokumenten beschrieben.

Die durch Glycosidierung erhaltenen funktionalisierten Alkoholgemische zeigen eine sehr gute Oberflächenaktivität und können als nichtionische Tensid in einer Vielzahl von Anwendungsbereichen vorteilhaft eingesetzt werden.

Die Sulfatierung oder Phosphatierung der zuvor beschriebenen Alkoholgemische oder alkoxilierten Alkoholgemische erfolgt durch Umsetzung mit Schwefelsäure oder Schwefelsäurederivaten zu sauren Alkylsulfaten oder Alkylethersulfaten oder durch Umsetzung mit Phosphorsäure oder oder Phosphorsäurederivaten zu sauren Alkylphosphaten oder Alkyletherphosphaten.

Geeignete Verfahren zur Sulfatierung von Alkoholen sind die üblichen, dem Fachmann bekannten, wie sie z. B. in der US 3,462,525, US 3,420,875 oder US 3,524,864 beschrieben werden, worauf hier in vollem Umfang Bezug genommen wird. Geeignete Verfahren zur Sulfatierung sind auch in Ullmanns Encyclopedia of Industrial Chemistry, 5. Aufl. Bd A25 (1994), S. 779-783 und der dort zitierten Literatur beschrieben.

Wird zur Sulfatierung der erfindungsgemäßen Alkoholgemische Schwefelsäure eingesetzt, so ist diese vorzugsweise 75 bis 100 gew.-%ig, insbesondere 85 bis 98 gew.-%ig. Derartige Schwefelsäure ist unter den Bezeichnungen konzentrierte Schwefelsäure und Monohydrat erhältlich.

Gewünschtenfalls kann zur Sulfatierung mit Schwefelsäure ein Lösungs- oder Verdünnungsmittel eingesetzt werden. Geeignete Lösungsmittel sind z. B. solche, die mit Wasser ein Azeotrop bilden, wie z. B. Toluol.

Nach einer geeigneten Ausführungsform zur Herstellung sulfatierter Alkoholgemische wird das Alkoholgemisch in einem Reaktionsgefäß vorgelegt und das Sulfatierungsmittel unter ständigem Durchmischen zugegeben. Zur Erzielung einer möglichst vollständigen Veresterung des Alkoholgemischs beträgt das Molmengenverhältnis von Alkanol zu Sulfatierungsmittel bevorzugt etwa 1:1 bis 1:1,5, insbesondere 1:1 bis 1:1,2. Gewünschtenfalls kann das Sulfatierungsmittel auch in einem molaren Unterschuss eingesetzt werden, z. B. bei der Sulfatierung alkoxilierter Alkoholgemische, wenn Gemische aus nichtionischen und anionischen grenzflächenaktiven Verbindungen hergestellt werden sollen. Die Sulfatierung erfolgt bevorzugt bei einer Temperatur im Bereich von Umgebungstemperatur bis 80 °C, insbesondere 40 bis 75 °C.

Weitere geeignete Sulfatierungsmittel sind z. B. Schwefeltrioxid, Schwefeltrioxid-Komplexe, Lösungen von Schwefeltrioxid in Schwefelsäure (Oleum), Chlorsulfonsäure, Sulfurylchlorid, Amidosulfonsäure etc. Bei Einsatz von Schwefeltrioxid als Sulfatierungsmittel kann die Umsetzung vorteilhaft in einem Fallfilmverdampfer, bevorzugt im Gegenstrom, durchgeführt werden. Dabei kann die Umsetzung diskontinuierlich oder kontinuierlich erfolgen.

Die Aufarbeitung der bei der Sulfatierung entstehenden Reaktionsgemische erfolgt nach üblichen, dem Fachmann bekannten Verfahren. Dazu zählt z. B. die Neutralisierung, Abtrennung gegebenenfalls eingesetzter Lösungsmittel etc.

Die Phosphatierung der zuvor beschriebenen Alkoholgemische und alkoxilierten Alkoholgemische erfolgt im Allgemeinen in analoger Weise zur Sulfatierung.

Geeignete Verfahren zur Phosphatierung von Alkoholen sind die üblichen, dem Fachmann bekannten, wie sie z. B. in Synthesis 1985, S. 449-488 beschrieben werden, worauf hier in vollem Umfang Bezug genommen wird.

Geeignete Phosphatierungsmittel sind z. B. Phosphorsäure, Polyphosphorsäure, Phosphorpentoxid, POCl₃ etc. Bei Einsatz von POCl₃ werden die verbleibenden Säurechloridfunktionen nach der Veresterung hydrolysiert.

Die durch Sulfatierung oder Phosphatierung erhaltenen funktionalisierten Alkoholgemische und deren Salze zeigen eine sehr gute Oberflächenaktivität und können als anionische Tenside in einer Vielzahl von Anwendungsbereichen vorteilhaft eingesetzt werden.

Ein weiterer Gegenstand der Erfindung sind die durch das zuvor beschriebene Verfahren erhältlichen funktionalisierten Alkoholgemische und deren Salze.

Ein weiterer Gegenstand der Erfindung ist die Verwendung der funktionalisierten Alkoholgemische, wie zuvor beschrieben, als Tenside, als oder in Dispergiermittel(n), Papierhilfsmittel(n), Textilhilfsmittel(n), Lederhilfsmittel(n), Schmutzlösungsmittel(n), Korrosionsinhibitoren, Hilfsmittel(n) für Dispersionen, Inkrustierungsinhibitoren, sowie in Produkten für die metallverarbeitende Industrie, in Produkten für Galvanoprozesse, in Lakken, Druckfarben, Pigmentzubereitungen, Pflanzenschutzformulierungen, in der Wasserbehandlung und in der kunststoffverarbeitenden Industrie.

Insbesondere die Alkoxilate zeigen vorteilhafte Eigenschaften. Aus den erfindungsgemäßen Alkoholen zugängliche C13-C₁₅-Alkoholalkoxilate ergeben bei der Messung der biologischen Abbaubarkeit (nach OECD 301 B) ein deutlich schnelleres Erreichen des pass levels von 60 % CO₂-Bildung als vergleichbare Alkoxilate. Da die erfindungsgemäßen Tenside vor allem in abwasserrelevanten Anwendungen, wie z. B. Waschmitteln und Reinigern für harte Oberflächen (hard surface cleaner), eingesetzt werden, ist dies ein entscheidender Vorteil.

Außerdem ist das Gelverhalten dieser erfindungsgemäßen C13-C₁₅-Alkoholalkoxilate überraschend. Es ist dem Fachmann bekannt, dass dieser Gelbereich von der Linearität des Alkohols abhängt. Diese Linearität ist im Rahmen dieser Anmeldung definiert als die Summe aus unverzweigten und niedrigverzweigten Alkoholen, worunter lineare sowie solche mit 2-Methyl- und 2-Ethyl-Struktur verstanden werden. Bei höherer Linearität resultieren Tenside, die ein ausgeprägteres Gelverhalten, also breitere Gelphasen bei der Verdünnung mit Wasser, aufweisen. Gemessen als Brookfield-Viskosität bei unterschiedlichen Konzentrationen in Wasser ergibt sich jedoch eine völlige Abwesenheit bzw. ein deutlich verkleinerter Gelbereich für die erfindungsgemäßen Tenside.

Dies bringt Vorteile vor allem in festen Wasch- und Reinigungsmitteln mit sich, unabhängig davon, ob diese Wasch- und Reinigungsmittel in Pulver-, Granulat-, Extrudat- oder Tablettenform vorliegen. Beim Auflösen dieser festen Produkte in Wasser ist die zu durchlaufende Gelphase deutlich kleiner und die Auflösung geht damit schneller vonstatten, was sich in einer längeren Einwirkzeit der vollen Tensidkonzentration auf das zu reinigende Gut positiv auswirkt. Die Reinigungsleistung wird dadurch erhöht.

Ein weiterer überraschender Effekt ist das geringe Schaumvermögen der Tenside auf Basis der erfindungsgemäßen C13-C₁₅-Alkohole. Es gilt allgemein, dass Tenside auf Basis linearer Alkohole stärker schäumen als Tenside auf Basis verzweigter Alkohole. Das mit den erfindungsgemäßen Tensiden gefundene Ergebnis widerspricht der Theorie.

Das geringere Schaumvermögen ist ebenfalls von Vorteil in vielen Wasch- und Reinigungsmitteln. So können geringere Mengen Schaumdämpfer, wie z. B. Silikone und Alkoholpropoxilate, eingesetzt werden. Dies ist vor allem bei festen und flüssigen Waschmitteln wichtig. Andererseits können diese Tenside in Bereichen eingesetzt werden, die aufgrund des Reinigungsverfahrens zu starkem Schäumen neigen, beispielsweise in Spritzreinigungsverfahren und in Hochdruckreinigern.

In Waschmitteln können die erfindungsgemäßen Tenside, vorzugsweise nichtionische C13-C₁₅-Alkoholalkoxilate, mit den üblichen, einem Fachmann bekannten Zusatzstoffen in Mengen von 0,1 bis 40 Gew.-%, vorzugsweise 0,5 bis 30 Gew.-%, meistbevorzugt 1 bis 20 Gew.-%, kombiniert werden. Beispiele für geeignete Zusatzstoffe umfassen:
- Builder und Cobuilder, beispielsweise Polyphosphate, Zeolithe, Polycarboxylate, Phosphonate, Citrate, Komplexbildner,
- ionische Tenside, beispielsweise Alkylbenzolsulfonate, α-Olefinsulfonate und andere Alkoholsulfate/-ethersulfate, auch die erfindungsgemäßen C₁₃-C₁₅-Alkoholsulfate/-ethersulfate, Sulfosuccinate,
- andere nichtionische Tenside, beispielsweise Alkylaminoalkoxylate und Alkylpolyglucoside, amphotere Tenside, z. B. Alkylaminoxide, Betaine, auch die erfindungsgemäßen C₁₃-C₁₅-Alkylpolyglucoside,
- optische Aufheller,
- Farbübertragungsinhibitoren, z. B. Polyvinylpyrrolidon,
- Stellmittel, z. B. Natriumsulfat, Magnesiumsulfat,
- Soil Release Agentien, z. B. Polyether/-ester, Carboxymethylcellulose,
- Inkrustierungsinhibitoren, z. B. Polyacrylate, Copolymere aus Acrylsäure und Maleinsäure,
- Bleichsysteme, bestehend aus Bleichmitteln, z. B. Perborat oder Percarbonat, plus Bleichaktivatoren, z. B. Tetraacetylethylendiamin, plus Bleichstabilisatoren,
- Parfüm,
- Schaumdämpfer, z. B. Silikonöle, Alkoholpropoxilate (vor allem in Flüssigwaschmitteln),
- Enzyme, z. B. Amylasen, Lipasen, Proteasen oder Carboxylasen,
- Alkalispender, z. B. Pentanatriummetasilicat oder Natriumcarbonat.

Weitere, einem Fachmann bekannte Bestandteile können ebenfalls enthalten sein.

Die pulverförmigen Waschmittel haben in der herkömmlichen Form eine durchschnittliches Schüttgewicht von ca. 450 g/l. Kompakt- oder Ultra-Kompaktwaschmittel weisen ein Schüttgewicht > 600 g/l auf. Diese gewinnen immer mehr an Bedeutung.

Flüssige Waschmittel können zusätzlich Lösungsmittel enthalten, z. B. Ethanol, Isopropanol, 1,2-Propylenglykol oder Butylenglykol.

Gelförmige Waschmittel enthalten zusätzlich Verdicker, wie z. B. Polysaccharide und schwachvernetzte Polycarboxylate (z. B. die Carbopol®-Marken der BF Goodrich).

Bei tablettenförmigen Waschmitteln werden weitere Zusatzstoffe benötigt. Dies sind beispielsweise Tablettierhilfsmittel, z. B. Polyethylenglykole mit Molmassen > 1000 g/mol oder Polymerdispersionen. Benötigt werden auch Tablettensprengmittel, z. B. Cellulosederivate, vernetztes Polyvinylpyrrolidon, vernetzte Polyacrylate oder Kombinationen aus Säuren, z. B. Zitronensäure, mit Natriumcarbonat.

In Reinigern für harte Oberflächen, z. B. sauren Reinigern, alkalischen Reinigern, Neutralreinigern, Maschinengeschirrreinigung, Metallentfettung, Glasreiniger, Fußbodenreiniger, um nur einige zu nennen, werden die erfindungsgemäßen nichtionischen C13-C₁₅-Alkoholalkoxilate kombiniert mit den nachfolgend aufgeführten, in Mengen von 0,01 bis 40 Gew.-%, vorzugsweise 0,1 bis 20 Gew.%, vorhandenen Zusatzstoffen.
- ionischen Tensiden, wie z. B. den erfindungsgemäßen C₁₃-C₁₅-Alkoholsulfat/-ethersulfaten, Alkylbenzolsulfonaten, α-Olefinsulfonaten, anderen Alkoholsulfaten/-ethersulfaten, Sulfosuccinaten
- anderen nichtionischen Tensiden, z. B. Alkylaminalkoxilaten und Alkylpolyglucosiden, auch den erfindungsgemäßen C₁₃-C₁₅-Alkylpolyglucosiden
- amphoteren Tensiden, z. B. Alkylaminoxiden, Betainen
- Buildern, z. B. Polyphosphaten, Polycarboxylaten, Phosphonaten, Komplexbildnern
- Dispergiermitteln, z. B. Naphthalinsulfonsäurekondensate, Polycarboxylate
- pH-regulierende Verbindungen, z. B. Alkalien wie NaOH, KOH oder Pentanatriummetasilikat oder Säuren, etwa Salzsäure, Phosphorsäure, Amidoschwefelsäure, Citronensäure
- Enzyme, z. B. Lipasen, Amylasen, Proteasen, Carboxylasen
- Parfüm
- Farbstoffe
- Biozide, z. B. Isothiazolinone, 2-Bromo-2-nitro-1,3-propandiol.
- Bleichsysteme, bestehend aus Bleichmitteln, z. B. Perborat, Percarbonat, plus Bleichaktivatoren, z. B. Tetraacetylethylendiamin, plus Bleichstabilisatoren
- Solubilisatoren, z. B. Cumolsulfonate, Toluolsulfonate, kurzkettige Fettsäuren, Phosphorsäurealkyl/-arylester
- Lösemittel, z. B. kurzkettige Alkyloligoglykole, Alkohole, wie Ethanol oder Propanol, aromatische Lösemittel, wie Toluol oder Xylol, N-Alkylpyrrolidone, Alkylencarbonate
- Verdicker, wie z. B. Polysaccharide und schwachvernetzte Polycarboxylate (z. B. die Carbopol®-Marken der BF Goodrich).

Diese Reiniger für harte Oberflächen sind gewöhnlich, aber nicht ausschließlich, wässrig und liegen in der Form von Mikroemulsionen, Emulsionen oder Lösungen vor.

Sollten sie in fester Form vorliegen, können zusätzlich Stellmittel wie oben beschrieben eingesetzt werden.

Bei tablettenförmigen Reinigern werden weitere Zusatzstoffe benötigt. Dies sind beispielsweise Tablettierhilfsmittel, z. B. Polyethylenglykole mit Molmassen > 1000 g/mol oder Polymerdispersionen. Benötigt werden auch Tablettensprengmittel, z. B. Cellulosederivate, vernetztes Polyvinylpyrrolidon, vernetzte Polyacrylate oder Kombinationen aus Säuren, z. B. Zitronensäure, mit Natriumcarbonat.

Die Tenside können auch mit Vorteil für eine Vielzahl anderer chemisch-technischer Prozesse eingesetzt werden, so generell in der metallverarbeitenden Industrie, beispielsweise in
- Kühlschmierstoffen,
- Härteölen,
- Hydraulikölemulsionen,
- Polierpasten,
- Formtrennmitteln,
- Ziehölen,
- Beizmitteln,
- Metallreinigern,
- Metalltrocknern.

Hierbei können die Tenside speziell in den Prozessen mit Vorteil eingesetzt werden, in denen es auf eine hohe thermische Stabilität ankommt.

Weiterhin können die Tenside bei der Herstellung und Verarbeitung von Textilien eingesetzt werden. Die Anwendung von Tensiden bei der Herstellung und Verarbeitung von Textilien ist außerordentlich vielseitig, sie erstreckt sich hauptsächlich auf die Gebiete
- Vorbehandlungsmittel von Fasern,
- Herstellung von Reyon-Fasern,
- Spinnpräparationen und Textilschmälzen,
- Färbehilfsmittel,
- Avivagen,
- Hydrophobiermittel,
- Hilfsmittel für den Druck,
- Antistatika,
- Beflockungs- und Beschichtungsmittel.

Weiterhin können die Tenside in der Leder-, Papier-, Druck-, Galvano- und Photoindustrie verwendet werden. Wichtige Anwendungsgebiete hierbei sind Lacke, Pigmente und Druckfarben. Eingesetzt werden Tenside in diesen Anwendungsgebieten sowohl in wässrigen als auch in nichtwässrigen Systemen. In nichtwässrigen Systemen dienen sie vor allem als Dispergierhilfsmittel, Antiabsetzmittel oder Verlaufhilfsmittel. Zudem ermöglichen Tenside die Herstellung von sogenannten High-Solids-Systemen, in denen sie neben der Stabilisierung der durch Emulsionspolymerisation oder -polykondensation hergestellten Bindemittel auf Kunststoffdispersionsbasis auch als Dispergierhilfsmittel oftmals eingesetzter organischer und anorganischer Pigmente dienen. Daneben verbessern sie die Hafteigenschaften dieser Anstrichmittel.

Weiterhin können die Tenside bei der Wasserbehandlung, beispielsweise bei der Abwasserreinigung, verwendet werden.

Weiterhin können die Tenside in Pflanzenschutz-Formulierungen eingesetzt werden.

weiterhin können die Verbindungen als Tenside oder Emulgatoren in der kunststoffherstellenden und kunststoffverarbeitenden Industrie verwendet werden. Hauptanwendungsgebiete bei der Kunststoffherstellung und -verarbeitung sind
- Herstellung von Kunststoffdispersionen,
- Herstellung von Perlpolymerisation,
- Herstellung von Schaumstoffen,
- Verwendung von grenzflächenaktiven Formtrennmitteln,
- Herstellung von Mikrokapseln,
- Verbesserung der Adhäsion zwischen Füll- und Kunststoffen,
- Zusätze zu Kunststoffdispersionen zur Erzielung besonderer Effekte wie Verschäumbarkeit, Füllstoffverträglichkeit oder Netzvermögen,
- Emulgatoren für nichtwässrige Systeme,
- Anfärben von Kunststoffen,
- Antistatische Ausrüstung von Kunststoffen,
- Klebstoffe.

Das erfindungsgemäße Verfahren wird nun in den nachfolgenden Beispielen näher erläutert. Dabei wurde der biologische Abbau gemäß den Richtlinien der OECD, hier spezielle OECD 301 B, durchgeführt. Die Schaumbildung wurde gemäß der DIN 53902 Blatt 1 durchgeführt bei einer Konzentration von 2 g/l. Der angegebene Wert entspricht dem V2-Wert in der DIN-Norm. Die Viskosität wurde mit einem Brookfield-Viskosimeter des Typs LVT gemessen. Vorher wurden die Tenside entsprechend mit Wasser verdünnt und auf Blasenfreiheit der Lösung geachtet. Bei einer Viskosität von > 10000 mPas geht man von einem Gel aus.

### Beispiele

### Beispiel 1

### Herstellung eines C13-C₁₅-Alkoholgemischs durch Rhodium-katalysierte Hydroformylierung und nachfolgende Hydrierung eines C₁₂-C₁₄-Olefingemischs

Ein technisches C12-C₁₄-α-Olefingemisch mit der in Tabelle I gegebenen Zusammensetzung wurde in einer kontinuierlich betriebenen Hydroformylierungsanlage hydroformyliert. Dabei wurden Olefingemisch und eine Katalysatorlösung in eine Kaskade aus zwei gerührten Autoklav-Reaktoren mit jeweils 1,6 Liter Flüssigvolumen eingespeist und dort bei einer Rhodiumkonzentration von ca. 20 mg/kg Reaktorinhalt bei einer Temperatur von 115 °C (erster Reaktor) und 135 °C (zweiter Reaktor) mit Synthesegas eines Mischungsverhältnisses von 1:1 CO/H₂ bei 280 bar umgesetzt. Der Katalysator enthält ein entsprechend Beispiel 4 der DE-A 198 01 437 hergestelltes Polyethylenimin einer mittleren Molmasse Mw von 460000, das mit ca. 60 Mol-% Laurinsäure, bezogen auf die enthaltenen Amingruppen, amidiert worden war und das dadurch homogen in der Katalysatorphase und in der Reaktionszone löslich war. Das molare Verhältnis von Stickstoff aus dem polymeren Liganden zu Rhodium betrug ca. 100. Der Austrag der Reaktoren wurde entspannt und das verbleibende Gas abgetrennt. Die Flüssigphase wurde in einen Verdampfer geleitet, in dem bei einer Temperatur von 170 °C und einem Druck von ca. 10 mbar ein Kopfstrom abgetrennt wurde.

Der Umsatz, bezogen auf das Gesamtolefin, betrug 98,3 %, die (Aldehyd+Alkohol)-Selektivität 98 %, die Paraffin-Selektivität 1 %, die Selektivität zu höhersiedenden Aldehyd-Kondensationsprodukten 1 %. Der Sumpfstrom enthielt den Rhodium-Katalysator und amidiertes Polyethylenimin und im Wesentlichen die hochsiedenden Kondensationsprodukten der Aldehyde und wurde in die Reaktionszone als Katalysatorlösung zurückgeführt. Der Kopfstrom des Verdampfers wurde in einer kontinuierlich betriebenen Hydrieranlage ohne weitere vorherige Aufarbeitung hydriert. Dabei wurde der Strom in einen mit einem Heterogenkatalysator (Cu-Katalysator auf Träger) bestückten Rohrreaktor in Rieselfahrweise bei 170 °C und einem Wasserstoffdruck von 280 bar unter Zugabe von 10 Gew.-% Wasser hydriert. Nach dem Entspannen und Abtrennen des Wasserstoffs wurde der Rohaustrag destillativ fraktioniert. Die Kohlenwasserstoffe wurden verworfen. Die C₁₃-C₁₅-Alkoholfraktionen wurden vollständig vereint und mittels Gaschromatographie mit internem Standard und Korrekturfaktoren deren Zusammensetzung bestimmt (Tabelle II).

**Tabelle I:**

| Zusammensetzung des eingesetzten C₁₂-C₁₄-α-Olefingemischs: | |
|---|---|
| | Gew.-% |
| C₁₂-Olefine | 67 |
| C₁₄-Olefine | 33 |
| α-Olefine | 88,6 |
| interne lineare Olefine | 2,5 |
| vinylidenverzweigte Olefine + sonstige Olefinisomere | 8,9 |

**Tabelle II:**

| Zusammensetzung des C13-C₁₅-Alkoholgemischs nach Hydrierung und Destillation: | |
|---|---|
| | Gew.-% |
| unverzweigte Alkohole | 50 |
| 2-Methyl-verzweigte Alkohole | 34 |
| 2-Ethyl-verzweigte Alkohole | 4 |
| höher verzweigte Alkohole | 12 |

### Beispiel 2 (Vergleichsbeispiel)

### C13-C₁₅-Alkohol durch cobaltkatalysierte Hydroformylierung eines C₁₂-C₁₄-Olefingemischs und nachfolgende Hydrierung

Das C12-C₁₄-α-Olefingemisch aus Beispiel 1 mit der in Tabelle I dargestellten Zusammensetzung wurde in der in Beispiel 1 gezeigten Weise hydroformyliert. Abweichend von Beispiel 1 wurde anstelle des Rhodium-Katalysators und des Polyethylenimin-Liganden als Hydroformylierungs-Katalysator Dicobaltoctacarbonyl ohne weiteren Liganden eingesetzt, in einer Menge von 2 g Cobalt berechnet auf 1000 g Reaktorinhalt. Um bei gleichem Olefinzulauf wie in Beispiel 1 den gleichen Olefinumsatz von 98 bis 99 % zu erzielen, war eine Reaktortemperatur von 140 °C in beiden Reaktoren erforderlich. Die (Aldehyd+Alkohol)-Selektivität betrug 94 %, die Paraffin-Selektivität 3 %, die Selektivität zu höhersiedenden Aldehyd-Kondensationsprodukten 3 %. Nach dem Entspannen der Reaktoren wurde der Austrag mit wässriger Essigsäurelösung unter Einleiten von Luftsauerstoff behandelt, bis das Cobaltcarbonyl zu Cobaltacetat oxidiert war. Die wässrige, cobalthaltige Phase wurde in einem Phasenscheider abgetrennt und die organische Phase entsprechend Beispiel 1 hydriert und destilliert. Die Zusammensetzung des C₁₃-C₁₅-Alkoholgemischs ist in Tabelle III zusammengestellt.

**Tabelle III:**

| Zusammensetzung des C₁₃-C₁₅-Alkoholgemischs nach Hydrierung und Destillation | |
|---|---|
| | Gew.-% |
| unverzweigte Alkohole | 58 |
| 1-Methyl-verzweigte- Alkohole | 17 |
| 1-Ethyl-verzweigte- Alkohole | 6 |
| andere Alkohole | 19 |

### Beispiele 3 - 6: Herstellung von Oxoalkoholethoxilaten

### Beispiel 3

### Herstellung eines C13-C₁₅-Oxoalkoholethoxilats mit 7 Mol Ethylenoxid

416 g C₁₃-C₁₅-Fettalkohol (aus Beispiel 1) werden mit 1,5 g NaOH in einen trockenen 2 1-Autoklaven eingefüllt. Der Autoklaveninhalt wird auf 150 °C erhitzt, dann werden 616 g Ethylenoxid unter Druck in den Autoklaven gepresst. Nachdem sich die gesamte Ethylenoxidmenge im Autoklaven befindet, wird der Autoklav für 30 Minuten bei 150 °C gehalten. Nach dem Abkühlen wird der Katalysator mit Essigsäure neutralisiert.

Das erhaltene Tensid weist einen Trübungspunkt von 77°C, gemessen 1%ig in in 10%iger Butyldiglykollösung nach DIN 53917, auf. Die Oberflächenspannung bei einer Konzentration von 1 g/l beträgt 27,9 mN/m, gemessen nach DIN 53914.

### Beispiel 4

### Herstellung eines C13-C₁₅-Oxoalkoholethoxilats mit 11 mol Ethylenoxid

312 g C13-C₁₅-Fettalkohol (aus Beispiel 1) werden mit 1,5 g NaOH in einen trockenen 2 1-Autoklaven eingefüllt. Der Autoklaveninhalt wird auf 150 °C erhitzt, dann werden 726 g Ethylenoxid unter Druck in den Autoklaven gepresst. Nachdem sich die gesamte Ethylenoxidmenge im Autoklaven befindet, wird der Autoklav für 30 Minuten bei 150 °C gehalten. Nach dem Abkühlen wird der Katalysator mit Essigsäure neutralisiert.

Das erhaltene Tensid weist einen Trübungspunkt von 85,8 °C, gemessen 1%ig in Wasser nach DIN 53917, auf. Die Oberflächenspannung bei einer Konzentration von 1 g/l beträgt 32,4 mN/m, gemessen nach DIN 53914.

### Beispiel 5 (Vergleichsbeispiel)

### Herstellung eines C13-C₁₅-Oxoalkoholethoxilats mit 7 mol Ethylenoxid

416 g C₁₃-C₁₅-Fettalkohol (aus Beispiel 2) werden mit 1,5 g NaOH in einen trockenen 2 1-Autoklaven eingefüllt. Der Autoklaveninhalt wird auf 150 °C erhitzt, dann werden 616 g Ethlyenoxid unter Druck in den Autoklaven gepresst. Nachdem sich die gesamte Ethylenoxidmenge im Autoklaven befindet, wird der Autoklav für 30 Minuten bei 150 °C gehalten. Nach dem Abkühlen wird der Katalysator mit Essigsäure neutralisiert.

Das erhaltene Tensid weist einen Trübungspunkt von 76,7 °C, gemessen 1%ig in 10%iger Butyldiglykollösung nach DIN 53917, auf. Die Oberflächenspannung bei einer Konzentration von 1 g/l beträgt 28 mN/m, gemessen nach DIN 53914.

### Beispiel 6 (Vergleichsbeispiel)

### Herstellung eines C13-C₁₅-Oxoalkoholethoxilats mit 11 Mol Ethylenoxid

312 g C₁₃-C₁₅-Fettalkohol (aus Beispiel 2) werden mit 1,5 g NaOH in einen trockenen 2 1-Autoklaven eingefüllt. Der Autoklaveninhalt wird auf 150 °C erhitzt, dann werden 726 g Ethylenoxid unter Druck in den Autoklaven gepresst. Nachdem sich die gesamte Ethylenoxidmenge im Autoklaven befindet, wird der Autoklav für 30 Minuten bei 150 °C gehalten. Nach dem Abkühlen wird der Katalysator mit Essigsäure neutralisiert.

Das erhaltene Tensid weist einen Trübungspunkt von 85,5 °C, gemessen 1%ig in Wasser nach DIN 53917, auf. Die Oberflächenspannung bei einer Konzentration von 1 g/l beträgt 31,5 mN/m, gemessen nach DIN 53914.

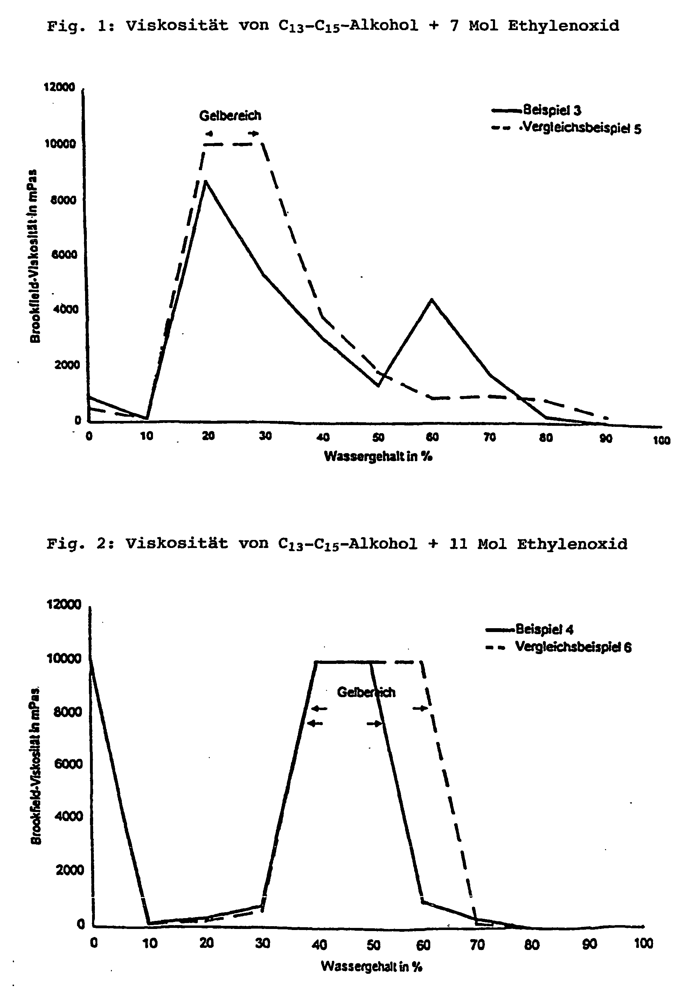

Von den in den Beispielen 3-6 erhaltenen Oxoalkoholethoxilaten wurden jeweils die biologische Abbaubarkeit, das Schaumvermögen und die Viskosität in Abhängigkeit vom wassergehalt bestimmt. Die Ergebnisse sind in den nachstehenden Tabellen IV-VI zusammengefasst. Die Figuren 1 und 2 zeigen die Viskosität in Abhängigkeit vom Wassergehalt.

**Tabelle IV:**

| Ergebnisse nach OECD 301 A/B-Kombitest | |
|---|---|
| | OECD 301 B Zeit der CO₂-Abnahme zwischen 10 und 60 % in Tagen |
| Beispiel 3 | 7,5 |
| Beispiel 4 | 8 |
| Beispiel 5 (Vergleich) | 9 |
| Beispiel 6 (Vergleich) | 8,5 |

**Tabelle V:**

| Schlagschaum nach DIN 53901 Bl. 1(Konzentration 2 g/l) | |
|---|---|
| | Schaumvolumen in ml |
| Beispiel 3 | 190 |
| Beispiel 4 | 270 |
| Beispiel 5 (Vergleich) | 250 |
| Beispiel 6 (Vergleich) | 400 |

**Tabelle VI:**

| Viskosität in mPas (Brookfield bei 23°C, D = 60 l/s) | | | | |
|---|---|---|---|---|
| Wassergehalt in % | Beispiel 3 | Beispiel 4 | Beispiel 5 (Vergleich) | Beispiel 6 (Vergleich) |
| 0 | 900 | Paste | 500 | Paste |
| 10 | 120 | 160 | 110 | 110 |
| 20 | 8700 | 320 | Gel | 200 |
| 30 | 5300 | 780 | Gel | 600 |
| 40 | 3100 | Gel | 3900 | Gel |
| 50 | 1400 | Gel | 1900 | Gel |
| 60 | 4500 | 1000 | 900 | Gel |
| 70 | 1800 | 330 | 1000 | 160 |
| 80 | 230 | 10 | 850 | 10 |
| 90 | 10 | 5 | 230 | 5 |

### Beispiel 7

### Herstellung eines C13-C₁₅-Oxoalkoholethoxilats mit 3 Mol Ethylenoxid

624 g des in Beispiel 1 hergestellten Alkohols werden mit 1,5 g NaOH in einen trockenen 2 1-Autoklaven eingefüllt. Der Autoklaveninhalt wird auf 150 °C erhitzt, dann werden 396 g Ethylenoxid unter Druck in den Autoklaven gepresst. Nachdem sich die gesamte Ethylenoxidmenge im Autoklaven befindet, wird der Autoklav für 30 Minuten bei 150 °C gehalten. Nach dem Abkühlen wird der Katalysator mit Schwefelsäure neutralisiert.

Das erhaltene Tensid weist einen Trübungspunkt von 46,3 °C, gemessen 1%ig in 10%iger Butyldiglykollösung nach DIN 53917, auf. Die Oberflächenspannung bei einer Konzentration von 1 g/l beträgt 28,6 mN/m, gemessen nach DIN 53914.

### Beispiel 8

### Herstellung eines Alkylphosphats

312 g des nach Beispiel 1 hergestellten Alkohols werden in einem Rührgefäß unter Stickstoff auf 60 °C erwärmt und langsam mit 125 g Polyphosphorsäure versetzt, wobei die Temperatur 65 °C nicht übersteigen sollte. Gegen Ende der Zugabe wird die Temperatur auf 70 °C erhöht und eine Stunde bei dieser Temperatur gerührt.

Die Oberflächenspannung bei einer Konzentration von 1 g/l beträgt 37,1 mN/m, gemessen nach DIN 53914.

### Beispiel 9

### Herstellung eines Alkyletherphosphats

510 g des in Beispiel 7 hergestellten C₁₃-C₁₅-Alkoholethoxilats werden in einem Rührgefäß unter Stickstoff auf 60 °C erwärmt und langsam mit 125 g Polyphosphorsäure versetzt. Dabei sollte die Temperatur 65 °C nicht übersteigen. Gegen Ende der Zugabe wird die Temperatur auf 70 °C erhöht und eine Stunde bei dieser Temperatur gerührt.

Die Oberflächenspannung bei einer Konzentration von 1 g/l beträgt 36,1 mN/m, gemessen nach DIN 53914.

### Beispiel 10

### Herstellung eines Alkylsulfats

208 g des nach Beispiel 1 hergestellten Alkohols werden in einem Rührgefäß unter Stickstoff langsam mit 132 g Chlorschwefelsäure versetzt. Dabei sollte die Temperatur 30°C nicht übersteigen. Diese Mischung wird in eine Lösung von 45 g NaOH in 710 ml Wasser gegeben.

Die Oberflächenspannung bei einer Konzentration von 1 g/l beträgt 31,8 mN/m, gemessen nach DIN 53914.

### Beispiel 11

### Herstellung eines Alkylethersulfats

510 g des in Beispiel 7 hergestellten C₁₃-C₁₅-Alkoholethoxilats werden in einem Rührgefäß unter Stickstoff langsam mit 198 g Chlorschwefelsäure versetzt. Dabei sollte die Temperatur 30 °C nicht übersteigen. Diese Mischung wird in eine Lösung von 67 g NaOH in 1500 ml Wasser gegeben.

Die Oberflächenspannung bei einer Konzentration von 1 g/l beträgt 34,2 mN/m, gemessen nach DIN 53914.

### Beispiel 12

### Herstellung eines C13-C₁₅-Alkoholalkoxylats

212 g des in Beispiel 1 hergestellten Alkohols werden mit 0,2 g KOH in einen trockenen 2 1-Autoklaven eingefüllt. Der Autoklaveninhalt wird auf 130 °C bis 140 °C erhitzt und 290 g Propylenoxid unter Druck in den Autoklaven gepresst. Nachdem sich die gesamte Propylenoxidmenge im Autoklaven befindet, wird der Autoklav für 120 Minuten bei 130 °C bis 140 °C gehalten. Anschließend werden bei 110 bis 120 °C 132 g Ethlyenoxid unter Druck in den Autoklaven gepresst und dieser 60 Minuten bei dieser Temperatur gehalten. Nach dem Abkühlen wird der Katalysator mit Essigsäure neutralisiert.

Das erhaltene Tensid weist einen Trübungspunkt von 44 °C, gemessen 1%ig in 10%iger Butyldiglykollösung nach DIN 53917, auf. Die Oberflächenspannung bei einer Konzentration von 1 g/l beträgt 31,2 mN/m, gemessen nach DIN 53914.

### Beispiel 13

### Herstellung eines C13-C₁₅ Alkoholalkoxylats

318 g des in Beispiel 1 hergestellten Alkohols werden mit 0,3 g KOH in einen trockenen 2 1-Autoklaven eingefüllt. Der Autoklaveninhalt wird auf 110 °C bis 120 °C erhitzt und 660 g Ethylenoxid unter Druck in den Autoklaven gepresst. Nachdem sich die gesamte Propylenoxidmenge im Autoklaven befindet, wird der Autoklav für 60 Minuten bei 110 °C bis 120 °C gehalten. Anschließend werden bei 140 bis 150 °C 200 g Butylenoxid unter Druck in den Autoklaven gepresst und dieser 150 Minuten bei dieser Temperatur gehalten. Nach dem Abkühlen wird der Katalysator mit Essigsäure neutralisiert.

Das erhaltene Tensid weist einen Trübungspunkt von 36 °C, gemessen 1%ig in Wasser nach DIN 53917, auf. Die Oberflächenspannung bei einer Konzentration von 1 g/l beträgt 30,4 mN/m, gemessen nach DIN 53914.

## Patentansprüche

1. Alkoholgemisch, das im Wesentlichen Alkohole mit 13 und 15 Kohlenstoffatomen enthält, bei dem der Anteil aus der Summe an C₁₃- und C₁₅-Alkoholen bei mindestens 95 Gew.-% liegt und wobei bezogen auf den Gesamtalkoholgehalt mindestens 87 Gew.-% der Alkohole ausgewählt sind unter linearen, 2-Methyl-verzweigten und 2-Ethyl-verzweigten Alkoholen und wobei das Gemisch 40 bis 60 Gew.-% lineare Alkohole, 30 bis 40 Gew.-% 2-Methyl-verzweigte Alkohole und 2 bis 7 Gew.-% 2-Ethyl-verzweigte Alkohole enthält.

2. Gemisch nach Anspruch 1, wobei das Verhältnis von Alkoholen mit 13 zu Alkoholen mit 15 Kohlenstoffatomen in einem Bereich von 90:10 bis 50:50 Gew.-%, bevorzugt 70:30 bis 60:40 Gew.-%, liegt.

3. Verfahren zur Herstellung von Alkoholgemischen nach einem der Ansprüche 1 oder 2, wobei man:
a) ein Monoolefingemisch bereitstellt, das im Wesentlichen Olefine mit 12 und 14 Kohlenstoffatomen enthält und das lineare α-Olefine und einen Anteil von 5 bis 20 Gew.-% an davon verschiedenen Olefinen aufweist, und
b) das Monoolefingemisch durch Umsetzung mit Kohlenmonoxid und Wasserstoff in Gegenwart eines Rhodiumkatalysators hydroformyliert und hydriert.

4. Verfahren nach Anspruch 3, wobei das Monoolefingemisch 85 bis 95 Gew.-% lineare α-Olefine, 1 bis 5 Gew.-% lineare interne Olefine, 5 bis 10 Gew.-% vinylidenverzweigte Olefine und gegebenenfalls bis zu 5 Gew.-% davon verschiedene sonstige Olefinisomere, bezogen auf den Gesamtolefingehalt, aufweist.

5. Verfahren nach einem der Ansprüche 3 oder 4, wobei der in Schritt b) eingesetzte Katalysator wenigstens einen Liganden aufweist, der ausgewählt ist unter zur Komplexbildung befähigten Verbindungen mit Carbonyl-, Carboxylat-, Hydrid-, Sulfat-, Nitrat-, stickstoffhaltigen und/oder phosphorhaltigen Gruppen, wobei die phosphorhaltige Gruppe höchstens einen Arylrest in Einfachbindung zum Phosphoratom aufweist.

6. Verfahren nach Anspruch 5, wobei der in Schritt b) eingesetzte Katalysator als Liganden wenigstens ein Phosphacyclohexan der allgemeinen Formeln I und II 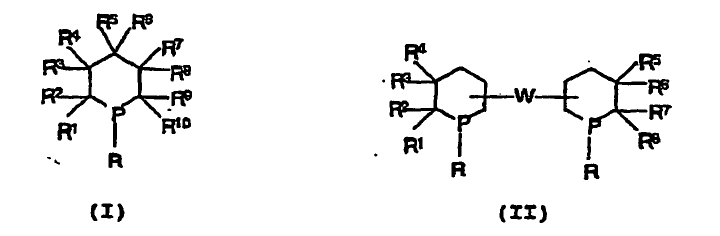 aufweist, mit der Bedeutung
R
Wasserstoff, C₁₋₁₀₀-Alkyl, Cycloalkyl, Heterocycloalkyl, C₇₋₂₀-Aralkyl, C₇₋₂₀-Alkaryl, C₆₋₁₂-Aryl, Betaryl, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR', W'((CH₂)₄O)ₓR' oder W'COR',
wobei in der Formel II die Reste R auch anstelle von oder zusätzlich zu der Gruppe W zusammen eine Brücke mit 1 bis 20 Kohlenstoffatomen bilden können, die Bestandteil einer cyclischen oder aromatischen Gruppe sein kann und durch Heteroatome unterbrochen sein kann,
wobei in der Formel II die Reste R auch anstelle von oder zusätzlich zu der Gruppe W zusammen für eine Polyoxyalkylen- oder Polyalkylenimin-Brücke mit mindestens 21 Kohlenstoffatomen stehen können,
R¹ bis R¹⁰
unabhängig voneinander Wasserstoff, C₁₋₂₀-Alkyl, C₇₋₂₀-Aralkyl, C₇₋₂₀-Alkaryl, C₆₋₁₂-Aryl, wobei eines oder mehrere Kohlenstoffatome durch Heteroatome ersetzt sein können, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR', W'((CH₂)₄O)ₓR', W'Halogen, W'NO₂, W'COR' oder W'CN,
wobei in den Resten R und R¹ bis R¹⁰ ein oder mehrere Wasserstoffatome durch Fluor ersetzt sein können,
W und W' unabhängig voneinander Einfachbindungen oder Brücken mit 1 bis 20 Kohlenstoffatomen, die Bestandteil einer cyclischen oder aromatischen Gruppe sein können und durch Heteroatome unterbrochen sein können,
wobei W auch für eine Polyoxyalkylen- oder Polyalkylenimin-Brücke mit mindestens 21 Kohlenstoffatomen stehen kann,
R' Wasserstoff, C₁₋₂₀-Alkyl, Carbonylalkyl, Cycloalkyl oder Aryl,
M⁺ Kationäquivalent,
X⁻ Anionäquivalent,
x Zahl von 1 bis 240,
wobei zwei geminale Reste R¹ bis R¹⁰ eine Oxo-Gruppe bilden können und einer oder mehrere der Reste R und R¹ bis R¹⁰ eine zusätzliche, zur Koordination befähigte dreibindige Phosphor- oder Stickstoffgruppierung aufweisen können,
wobei jeweils zwei vicinale Reste zu anellierten aliphatischen oder aromatischen Ringen verbunden sein können,
wobei zwei vicinale Reste R¹ bis R¹⁰ auch für eine chemische Bindung stehen können,
wobei in den Verbindungen der allgemeinen Formel II auch zwei oder mehr Brücken W vorliegen können und wobei die nicht an die Brücke(n) W gebundenen Atom der Phosphacyclohexanringe auch wie für R¹ bis R¹⁰ definiert substituiert sein können,
wobei in den Verbindungen der Formel I auch einer der Reste R oder R¹ bis R¹⁰ und in den Verbindungen der Formel II auch einer der Reste R oder R¹ bis R⁸ oder die beiden Reste R gemeinsam oder eine Gruppe W auch für einen Polymerrest mit einem zahlenmittleren Molekulargewicht im Bereich von 500 bis 50000 stehen kann, aufgebaut aus Wiederholungseinheiten, die sich von Monomeren ableiten, die ausgewählt sind unter Mono- und Diolefinen, Vinylaromaten, Estern α,β-ethylenisch ungesättigter Mono- und Dicarbonsäuren mit C₁-C₃₀-Alkanolen, N-Vinylamiden, N-Vinyllactamen, unter Ringöffnung polymerisierbaren heterocyclischen Verbindungen und Mischungen davon.

7. Verfahren nach Anspruch 6, wobei die Verbindung der allgemeinen Formel I ausgewählt ist aus Verbindungen der allgemeinen Formel III 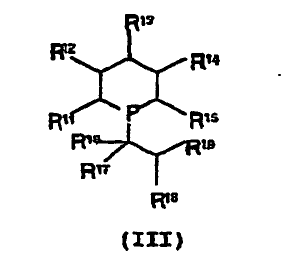 mit der Bedeutung:
R¹¹ bis R¹⁹ unabhängig voneinander Wasserstoff, C₁₋₂₀-Alkyl, C₇₋₂₀-Aralkyl, C₇₋₂₀-Alkaryl, C₆₋₁₂-Aryl, wobei eines oder mehrere Kohlenstoffatome durch Heteroatome ersetzt sein können, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR',
wobei jeweils zwei vicinale Reste R¹¹ bis R¹⁵ und/oder R¹⁷ und R¹⁸ und/oder R¹⁶ und R¹⁷ und/oder R¹⁶ und R¹⁹ und/oder R¹⁸ und R¹⁹ zu Ringen verbunden sein können,
W' Einfachbindung oder Brücke mit 1 bis 20 Kohlenstoffatomen, die Bestandteil einer cyclischen oder aromatischen Gruppe sein kann,
R' Wasserstoff oder C₁₋₆-Alkyl,
M⁺ Kation,
X⁻ Anion,
x Zahl von 1 bis 240,
wobei einer oder mehrere der Reste R¹¹ bis R¹⁹ eine zusätzliche, zur Koordination befähigte dreibindige Phosphor- oder Stickstoffgruppierung aufweisen können,
wobei R¹⁸ auch -W'-CR^{20'}=CR^{21'}R²², wobei R²⁰, R²¹, R²² wie vorstehend für R¹¹ bis R¹⁹ definiert sind, bedeuten kann.

8. Verfahren nach Anspruch 6, wobei die Verbindungen der Formeln I und II ausgewählt sind unter Verbindungen der allgemeinen Formeln 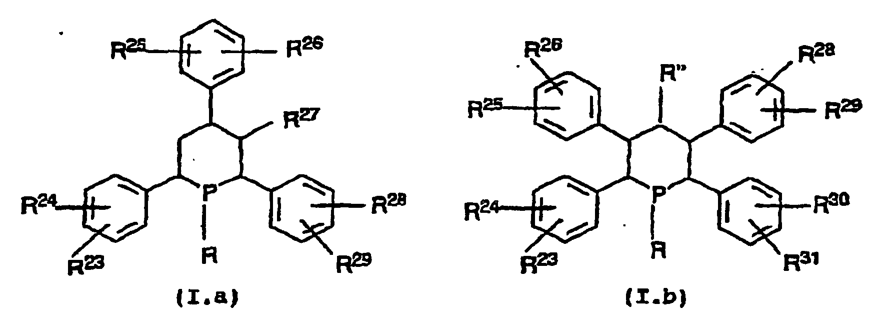 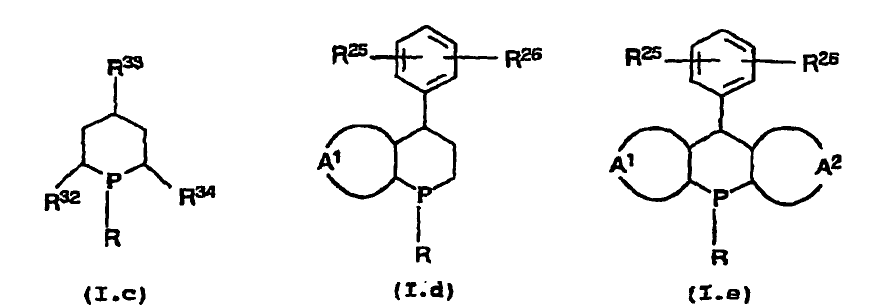 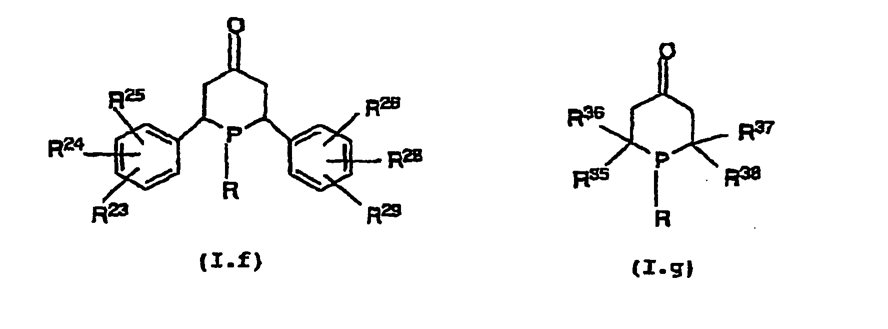 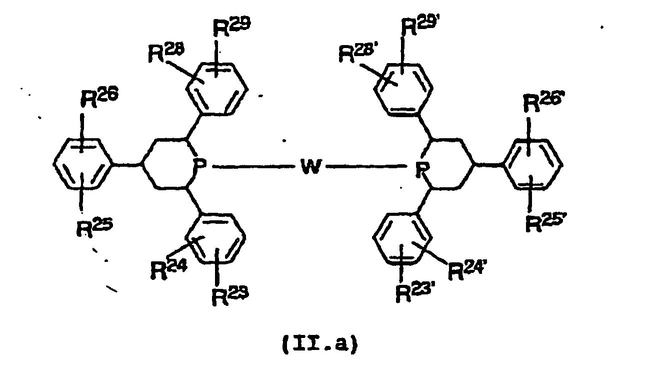 worin
R für C₁₋₂₀-Alkyl, Cycloalkyl, C₆₋₁₂-Aryl, W'(CHR'CH₂O)ₓR', W'((CH₂)₄O)ₓR' oder einen Polymerrest mit einem zahlenmittleren Molekulargewicht im Bereich von 500 bis 50000, der aufgebaut ist aus Ethylen und/oder Butadien, steht, wobei
W' für eine Einfachbindung oder C₁₋₄-Alkylen steht, und
R' für Wasserstoff oder C₁₋₂₀-Alkyl steht,
x für eine ganze Zahl von 1 bis 240 steht,
R²³, R^{23'}, R²⁴, R^{24'}, R²⁵, R^{25'}, R²⁶, R^{26'}, R²⁸, R^{28'}, R²⁹, R^{29'}, R³⁰ und R³¹ unabhängig voneinander für Wasserstoff, Alkyl, Alkoxy, Carboxyl, Carboxylat, Trifluormethyl, -SO₃H, Sulfonat, NE¹E² oder Alkylen-NE¹E² stehen, wobei E¹ und E² unabhängig voneinander für Wasserstoff, Alkyl oder Cycloalkyl stehen,
R²⁷ für Wasserstoff, Alkyl, Cycloalkyl, Aryl oder Aralkyl steht,
R³², R³³, R³⁴, R³⁵, R³⁶, R³⁷ und R³⁸ unabhängig voneinander für Alkyl oder cycloalkyl stehen,
R'' für Wasserstoff oder Phenyl steht,
A¹ und A² zusammen mit den benachbarten Kohlenstoffatomen des Phosphacyclohexans, an die sie gebunden wird, für ein anelliertes Ringsystem mit je 1 oder 2 weiteren Ringen stehen,
W für eine Brücke mit 1 bis 20 Kohlenstoffatomen steht, die durch Heteroatome unterbrochen sein kann.

9. Verfahren nach Anspruch 5, wobei der in Schritt b) eingesetzte Katalysator als Liganden wenigstens ein Polyamin aufweist, das im Wesentlichen nicht wasserlöslich ist, ein mittleres Molekulargewicht von > 1000 Dalton und mindestens 10 Stickstoffatome aufweist, vorzugsweise ein derivatisiertes Polyamin oder ein Polyethylenimin, das im wesentlichen aus Einheiten der Formel (IV) 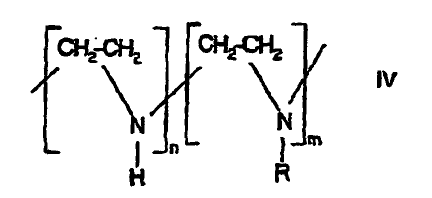 besteht, wobei die Summe aus m + n mindestens 10 und das Verhältnis aus m/m+n 0,01 bis 1 beträgt und R identische oder verschiedene Alkyl-, Cycloalkyl-, Aryl-, Aralkyl-, Alkylcaxbonylreste mit bis zu 30 C-Atomen oder Hydroxyalkyl(poly)oxyalkylenreste mit bis zu 500 Alkylenoxy-Einbeiten bedeutet.

10. Verfahren zur Herstellung funktionalisierter Alkoholgemische, wobei man ein Alkoholgemisch nach einem der Ansprüche 1 oder 2 einer Alkoxilierung, Glycosidierung, Sulfatierung, Phosphatierung, Alkoxilierung und nachfolgender Sulfatierung oder Alkoxilierung und nachfolgender Phosphatierung unterwirft.

11. Funktionalisiertes Alkoholgemisch, erhältlich durch ein Verfahren nach Anspruch 10.

12. Verwendung der funktionaklieierten Alkoholgemische nach Anspruch 11 als Tenside, als oder in Dispergiermittel(n), Papierhilfsmittel(n), Textilhilfsmittel(n), Lederhilfsmittel(n), Schmutzlösungsmittel(n), Korrosionsinhibitoren, Hilfsmittel(n) für Dispersionen, Inkrustierungsinhibitoren, sowie in Produkten für die metallverarbeitende Industrie, in Produkten für Galvanoprozesse, in Lacken, Druckfarben, Pigmentzubereitungen, Pflanzenschutzformulierungen, in der Wasserbehandlung und in der kunststoffverarbeitenden Industrie.

## Claims

1. An alcohol mixture substantively comprising alcohols having 13 or 15 carbon atoms, in which the proportion made up of all of the C₁₃ and C₁₅ alcohols is at least 95% by weight and
where, based on the total alcohol content, at least 87% by weight of the alcohols have been selected among linear, 2-methyl-branched, and 2-ethyl-branched alcohols and where the mixture comprises from 40 to 60% by weight of linear alcohols, from 30 to 40% by weight of 2-methyl-branched alcohols and from 2 to 7% by weight of 2-ethyl-branched alcohols.

2. The mixture according to claim 1, where the ratio of alcohols having 13 carbon atoms to alcohols having 15 carbon atoms is in the range from 90:10 to 50:50% by weight, preferably from 70:30 to 60:40% by weight.

3. A process for preparing alcohol mixtures according to claim 1 or 2, where:
a) a monoolefin mixture is prepared, substantively comprising olefins having 12 or 14 carbon atoms and having linear α-olefins and from 5 to 20% by weight of olefins other than these, and
b) the monoolefin mixture is hydroformylated by reaction with carbon monoxide and hydrogen in the presence of a rhodium catalyst, and is hydrogenated.

4. The process according to claim 3, where the monoolefin mixture has, based on the total olefin content, from 85 to 95% by weight of linear α-olefins, from 1 to 5% by weight of linear non-terminal olefins, from 5 to 10% by weight of vinylidene-branched olefins, and, where appropriate, up to 5% by weight of other olefin isomers differing therefrom.

5. The process according to claim 3 or 4, where the catalyst used in step b) has at least one ligand selected among compounds capable of forming complexes and having carbonyl, carboxylate, hydride, sulfate, or nitrate groups, or having nitrogen-containing and/or phosphorus-containing groups, where the phosphorus-containing group has no more than one aryl radical with single bonding to the phosphorus atom.

6. The process according to claim 5, where the catalyst used in 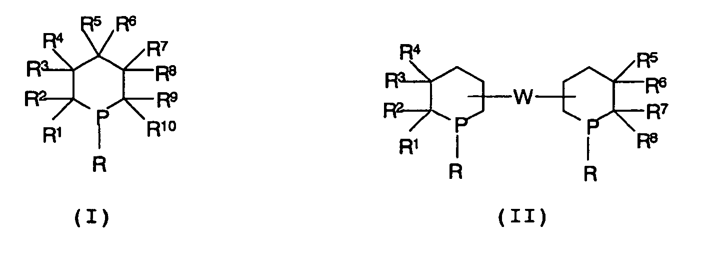 where
R is hydrogen, C₁₋₁₀₀-alkyl, cycloalkyl, heterocycloalkyl, C₇₋₂₀-aralkyl, C₇₋₂₀-alkaryl, C₆₋₁₂-aryl, hetaryl, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR', W'((CH₂)₄O)ₓR', or W'COR',
where in formula II the radicals R may also, instead of or in addition to the group W, together form a bridge having from 1 to 20 carbon atoms, which may be a constituent of a cyclic or aromatic group and may have interruption by heteroatoms,
and where in formula II the radicals R may also, instead of or in addition to the group W, together be a polyoxyalkylene bridge or polyalkyleneimine bridge having at least 21 carbon atoms,
R¹ to R¹⁰
independently of one another are hydrogen, C₁₋₂₀-alkyl, C₇₋₂₀-aralkyl, C₇₋₂₀-alkaryl, C₆₋₁₂-aryl, where one or more carbon atoms may have been replaced by heteroatoms, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR', W'((CH₂)₄O)ₓR', W'halogen, W'NO₂, W'COR' or W'CN,
where one or more hydrogen atoms in radicals R and R¹ to R¹⁰ may have been replaced by fluorine,
W and W' independently of one another are single bonds or bridges having from 1 to 20 carbon atoms, which may be a constituent of a cyclic or aromatic group and which may have interruption by heteroatoms,
where W may also be a polyoxyalkylene or polyalkylenimine bridge having at least 21 carbon atoms,
R' is hydrogen, C₁₋₂₀-alkyl, carbonylalkyl, cycloalkyl or aryl,
M⁺ is a cation equivalent,
X⁻ is an anion equivalent, and
x is an integer from 1 to 240,
where two geminal radicals R¹ to R¹⁰ may form an oxo group, and one or more of the radicals R and R¹ to R¹⁰ may have an additional triply bonded phosphorus-containing or nitrogen-containing group capable of coordination,
and where any two vicinal radicals may have bonding to give anellated, aliphatic or aromatic rings,
and where two vicinal radicals R¹ to R¹⁰ may also be a chemical bond,
and where in the compounds of the general formula II there may also be two or more bridges W present, and where those atoms of the phosphacyclohexane rings not bonded to the bridge(s) W may also have substitution as defined for R¹ to R¹⁰,
and where in the compounds of the formula I, one of the radicals R or R¹ to R¹⁰, and in the compounds of the formula II one of the radicals R or R¹ to R⁸ or the two radicals R together or a group W may also be a polymer radical with a number-average molecular weight in the range from 500 to 50 000, composed of repeat units which derive from monomers selected from mono- and diolefins, vinylaromatics, esters of α,β-ethylenically unsaturated mono- or dicarboxylic acids with C₁-C₃₀ alkanols, N-vinylamides, N-vinyllactams, heterocyclic compounds polymerizable with ring-opening, and mixtures of these.

7. The process according to claim 6, where the compound of the formula I has been selected from compounds of the formula III 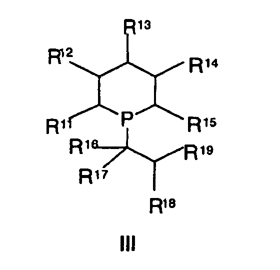 where:
R¹¹ to R¹⁹,
independently of one another, are hydrogen, C₁₋₂₀-alkyl, C₇₋₂₀-aralkyl, C₇₋₂₀-alkaryl, or C₆₋₁₂-aryl, where one or more carbon atoms may have replacement by heteroatoms, or W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR', W'(CH₂CH₂NR')ₓR',
where any two vicinal radicals R¹¹ to R¹⁵, and/or R¹⁷ and R¹⁸ and/or R¹⁶ and R¹⁷, and/or R¹⁶ and R¹⁹, and/or R¹⁸ and R¹⁹, may have bonding to give rings,
W' is a single bond or bridge having from 1 to 20 carbon atoms, which may be a constituent of a cyclic or aromatic group,
R' is a hydrogen or C₁₋₆-alkyl,
M⁺ is a cation,
X- is an anion, and
x is a number from 1 to 240,
where one or more of the radicals R¹¹ to R¹⁹ may have an additional triply bonded phosphorus-containing or nitrogen-containing group capable of coordination, and where R¹⁸ may also be -W'-CR²⁰=CR²¹R²², where R²⁰, R²¹ and R²² are as defined above for R¹¹ to R¹⁹.

8. The process according to claim 6, where the compounds of the formulae I and II have been selected from compounds of the formulae 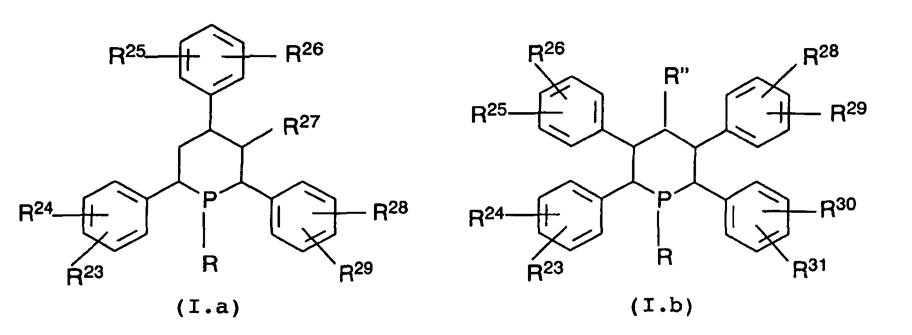 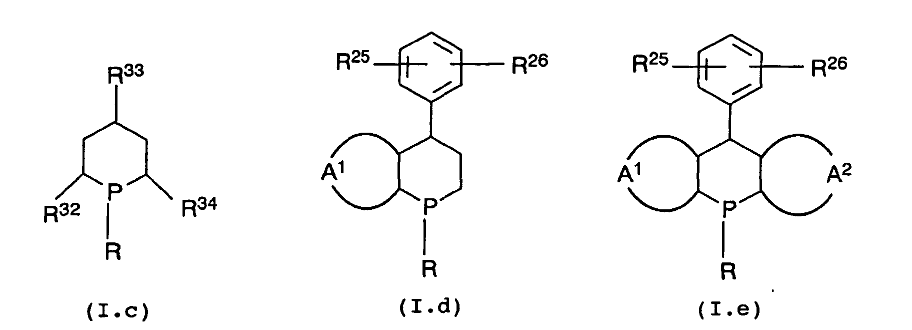 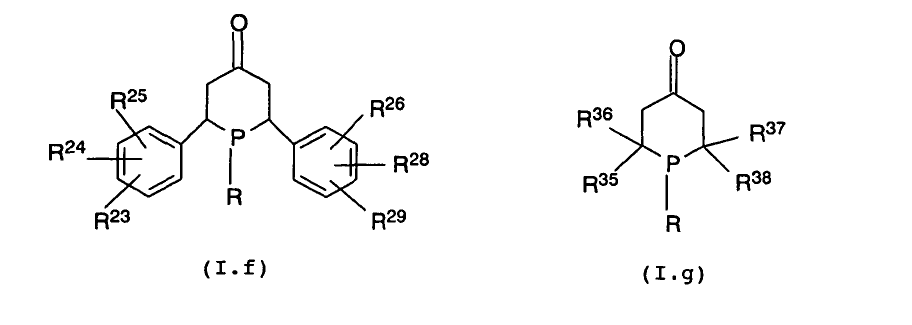 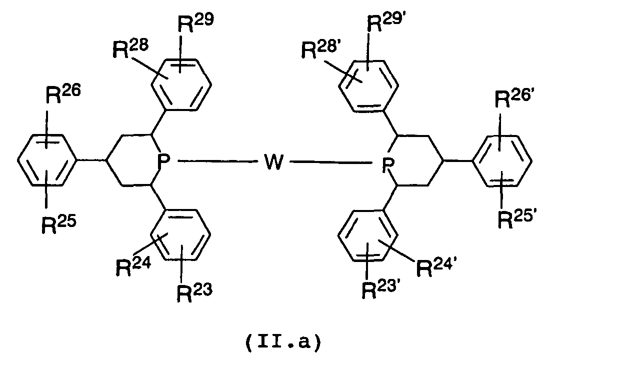 where
R is C₁₋₂₀-alkyl, cycloalkyl, C₆₋₁₂-aryl, W'(CHR'CH₂O)ₓR', W'((CH₂)₄O)ₓR', or a polymer radical with number-average molecular weight in the range from 500 to 50 000, composed of ethylene and/or butadiene, where
W' is a single bond or C₁₋₄-alkylene, and
R' is hydrogen or C₁₋₂₀-alkyl,
x is an integer from 1 to 240,
R²³, R^{23'}, R²⁴, R^{24'}, R²⁵, R^{25'}, R²⁶, R^{26'}, R²⁸, R^{28'}, R²⁹, R^{29'}, R³⁰, and R³¹, independently of one another, are hydrogen, alkyl, alkoxy, carboxy, carboxylate, trifluoromethyl, -SO₃H, sulfonate, NE¹E², or alkylene-NE¹E², where E¹ and E², independently of one another, are hydrogen, alkyl, or cycloalkyl,
R²⁷ is hydrogen, alkyl, cycloalkyl, aryl, or aralkyl,
R³², R³³, R³⁴, R³⁵, R³⁶, R³⁷, and R³⁸, independently of one another, are alkyl or cycloalkyl,
R'' is hydrogen or phenyl,
A¹ and A², together with those adjacent carbon atoms of the phosphacyclohexane to which they have been bonded, are an anellated ring system in each case having 1 or 2 further rings,
W is a bridge having from 1 to 20 carbon atoms, which may have interruption by heteroatoms.

9. The process according to claim 5, where the catalyst used in step b) has, as ligand, at least one polyamine which is substantively non-water-soluble and has an average molecular weight of > 1000 dalton, and has at least 10 nitrogen atoms, preferably a derivatized polyamine or a polyethyleneimine composed substantively of units of the formula (IV) 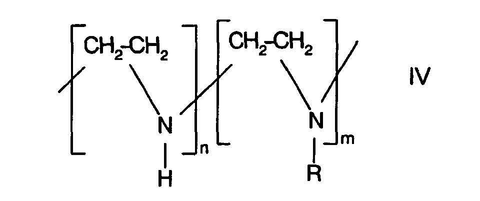 where m + n is at least 10 and the ratio m/m + n is from 0.01 to 1, and R is identical or different alkyl, cycloalkyl, aryl, aralkyl, or alkylcarbonyl radicals having up to 30 carbon atoms, or is hydroxyalkyl(poly)oxyalkylene radicals having up to 500 alkyleneoxy units.

10. A process for preparing functionalized alcohol mixtures, where an alcohol mixture according to claim 1 or 2 is subjected to alkoxylation, glycosidation, sulfation, phosphation, alkoxylation followed by sulfation, or alkoxylation followed by phosphation.

11. A functionalized alcohol mixture obtainable by a process according to claim 10.

12. The use of the functionalized alcohol mixtures according to claim 11 as surfactants, or as or in dispersants, paper auxiliaries, textile auxiliaries, leather auxiliaries, or detergents, corrosion inhibitors, auxiliaries for dispersions, or encrustation inhibitors, or else in products for the metalworking industry, in products for electroplating processes, in surface-coatings, in printing inks, in pigment preparations, in crop-protection formulations, in water treatment, or in the plastics processing industry.

## Revendications

1. Mélange d'alcools, qui contient essentiellement des alcools comportant 13 et 15 atomes de carbone, dans lequel la proportion de la somme des alcools en C₁₃ et C₁₅ est d'au moins 95 % en poids, au moins 87 % en poids des alcools, par rapport à la teneur globale en alcool, étant choisis parmi des alcools linéaires, ramifiés par du 2-méthyle et ramifiés par du 2-éthyle, le mélange contenant 40 à 60% en poids d'alcools linéaires, 30 à 40% en poids d'alcools ramifiés par du 2-méthyle et 2 à 7 % en poids d'alcools ramifiés par du 2-éthyle.

2. Mélange suivant la revendication 1, dans lequel le rapport entre alcools comportant 13 atomes de carbone et alcools en comportant 15 est d'un ordre de 90/10 à 50/50 % en poids, de préférence de 70/30 à 60/40 % en poids.

3. Procédé de préparation de mélanges d'alcools suivant l'une des revendications 1 et 2, dans lequel
a) on prépare un mélange monooléfinique qui contient essentiellement des oléfines comportant 12 et 14 atomes de carbone et qui présente des α-oléfines linéaires et une proportion de 5 à 20 % en poids d'oléfines différentes de celles-là, et
b) on hydroformyle et hydrogène le mélange monooléfinique par réaction avec du monoxyde de carbone et de l'hydrogène en présence d'un catalyseur à base de rhodium.

4. Procédé suivant la revendication 3, dans lequel le mélange monooléfinique présente 85 à 95 % en poids d'α-oléfines linéaires, 1 à 5 % en poids d'oléfines internes linéaires, 5 à 10 % en poids d'oléfines ramifiées par du vinylidène et éventuellement jusqu'à 5 % en poids d'autres isomères oléfiniques différents de celles-là, par rapport à la teneur totale en oléfine.

5. Procédé suivant l'une des revendications 3 et 4, dans lequel le catalyseur mis en oeuvre dans l'étape b) présente au moins un ligand qui est choisi parmi des composés susceptibles de complexation et comportant des groupes carbonyle, carboxylate, hydrure, sulfate, nitrate, azotés et/ou phosphorés, le groupe phosphoré présentant au maximum un radical aryle en liaison simple par rapport à l'atome de phosphore.

6. Procédé suivant la revendication 5, dans lequel le catalyseur mis en oeuvre dans l'étape b) présente, comme ligand, au moins un phosphacyclohexane des formules générales I et II : 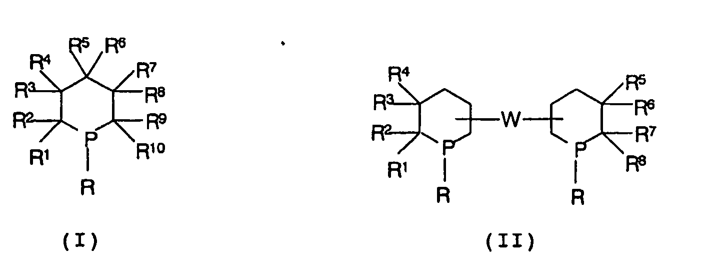 dans lesquelles
R représente
de l'hydrogène ou un groupe alkyle en C₁-C₁₀₀, cycloalkyle, hétérocycloalkyle, aralkyle en C₇-C₂₀, alkaryle en C₇-C₂₀, aryle en C₆-C₁₂, hétaryle, W'COO⁻M⁺, W'SO₃⁻M⁺, W' PO₃²⁻M⁺₂, W'NR'₃⁺X⁻, W'OR', W'NR'₂, W'COOR', W'SR', W'(CHR'CH₂O)ₓR', W' (CH₂NR')ₓR', W' (CH₂CH₂NR')ₓR', W' ((CH₂)₄O)ₓR' ou W'COR',
les radicaux R dans la formule II pouvant aussi, au lieu du groupe W ou en supplément de celui-ci, former conjointement un pont de 1 à 20 atomes de carbone, qui peut être un élément d'un groupe cyclique ou aromatique et être interrompu par des hétéroatomes,
les radicaux R dans la formule II pouvant aussi, au lieu du groupe W ou en supplément à celui-ci, représenter conjointement un pont de polyoxyalkylène ou de polyalkylènimine comportant au moins 21 atomes de carbone,
R¹ à R¹⁰ représentent
indépendamment l'un de l'autre de l'hydrogène ou un groupe alkyle en C₁-C₂₀, aralkyle en C₇-C₂₀, alkaryle en C₇-C₂₀, aryle en C₆-C₁₂, où un ou plusieurs atomes de carbone peuvent être remplacés par des hétéroatomes, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W' (CH₂NR')ₓR', W'(CH₂CH₂NR')ₓR', W'((CH₂)₄O)ₓR', W'halogène, W'NO₂, W'COR' ou W'CN,
un ou plusieurs atomes d'hydrogène dans les radicaux R et R¹ à R¹⁰ pouvant être remplacés par du fluor,
W et W' représentent indépendamment l'un de l'autre de simples liaisons ou des ponts comportant 1 à 20 atomes de carbone qui peuvent être un élément d'un groupe cyclique ou aromatique et être interrompus par des hétéroatomes,
W pouvant aussi représenter un pont de polyoxyalkylène ou de polyalkylènimine comportant au moins 21 atomes de carbone,
R' représente de l'hydrogène ou un groupe alkyle en C₁-C₂₀, carbonylalkyle, cycloalkyle ou aryle,
M⁺ représente un équivalent cationique,
X⁻ représente un équivalent anionique,
x est un nombre de 1 à 240,
deux radicaux géminés R¹ à R¹⁰ pouvant former un groupe oxo et un ou plusieurs des radicaux R et R¹ à R¹⁰ pouvant présenter un groupement supplémentaire d'azote ou de phosphore à trois liaisons capable de réaliser une coordination,
deux radicaux voisins pouvant être respectivement reliés à des noyaux aromatiques ou aliphatiques condensés,
deux radicaux voisins R¹ à R¹⁰ pouvant aussi représenter une liaison chimique,
deux ou plusieurs ponts W pouvant aussi se présenter dans les composés de la formule générale II et les atomes des noyaux de phosphacyclohexane qui ne sont pas liés au ou aux ponts W pouvant aussi être substitués comme défini pour R¹ à R¹⁰,
un des radicaux R ou R¹ à R¹⁰ dans les composés de la formule I et un des radicaux R ou R¹ à R⁸ ou les deux radicaux R conjointement ou un groupe W dans les composés de la formule II pouvant aussi représenter un radical de polymère présentant un poids moléculaire moyen numérique de l'ordre de 500 à 50 000, qui est constitué d'unités répétitives qui dérivent de monomères qui sont choisis parmi des monooléfines et des dioléfines, des substances vinyl-aromatiques, des esters d'acides monocarboxyliques et dicarboxyliques α,β-éthyléniquement insaturés avec des alcanols en C₁-C₃₀, des N-vinylamides, des N-vinyllactames, des composés hétérocycliques polymérisables par ouverture de noyau et leurs mélanges.

7. Procédé suivant la revendication 6, dans lequel le composé de la formule générale I est choisi parmi les composés de la formule générale III : 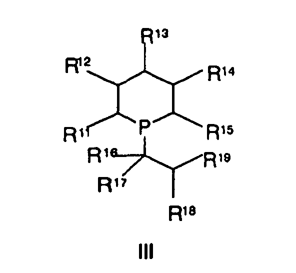 dans laquelle R¹¹ à R¹⁹ représentent
indépendamment l'un de l'autre de l'hydrogène ou un groupe alkyle en C₁-C₂₀, aralkyle en C₇-C₂₀, alkaryle en C₇-C₂₀, aryle en C₆-C₁₂, où un ou plusieurs atomes de carbone peuvent être remplacés par des hétéroatomes, W'COO⁻M⁺, W'SO₃⁻M⁺, W'PO₃²⁻M⁺₂, W'NR'₃⁺X⁻, W'OR', W'NR'₂, W'COOR', W'SR', W'(CHR'CH₂O)ₓR', W'(CH₂NR')ₓR' ou W'(CH₂CH₂NR')ₓR',
deux radicaux voisins R¹¹ à R¹⁵ et/ou R¹⁷ et R¹⁸ et/ou R¹⁶ et R¹⁷ et/ou R¹⁶ et R¹⁹ et/ou R¹⁸ et R¹⁹ pouvant respectivement être reliés en noyaux,
W' représente une simple liaison ou un pont comportant 1 à 20 atomes de carbone qui peut être un élément d'un groupe cyclique ou aromatique,
R' représente de l'hydrogène ou un groupe alkyle en C₁-C₆,
M⁺ est un cation,
X⁻ est un anion,
x est un nombre de 1 à 240,
un ou plusieurs des radicaux R¹¹ à R¹⁹ pouvant présenter un groupement supplémentaire d'azote ou de phosphore à trois liaisons capable de réaliser une coordination,
R¹⁸ pouvant aussi représenter -W'-CR²⁰'=CR²¹'R²², où R²⁰, R²¹, R²² sont comme définis précédemment pour R¹¹ à R¹⁹.

8. Procédé suivant la revendication 6, dans lequel les composés des formules I et II sont choisis parmi des composés des formules générales : 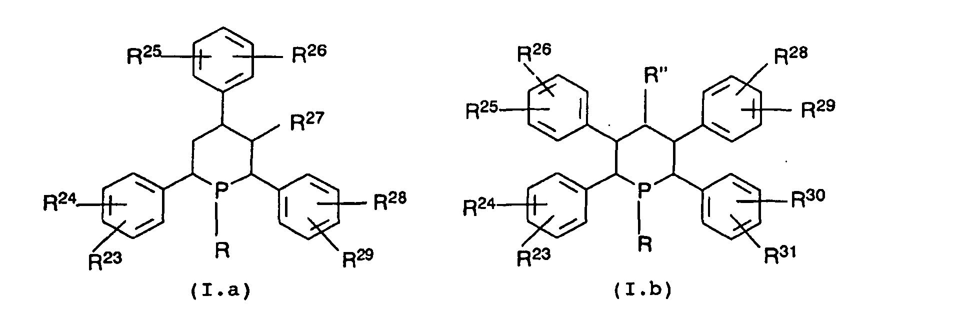 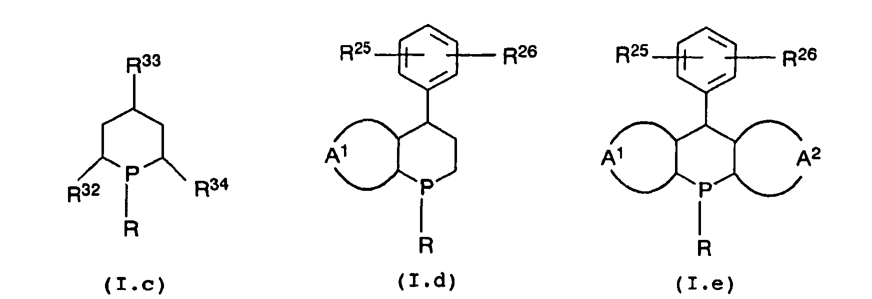 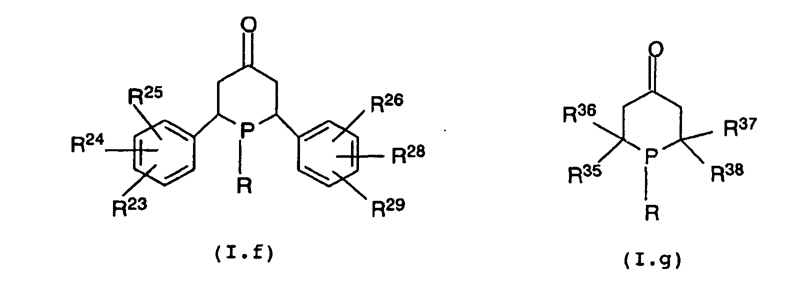 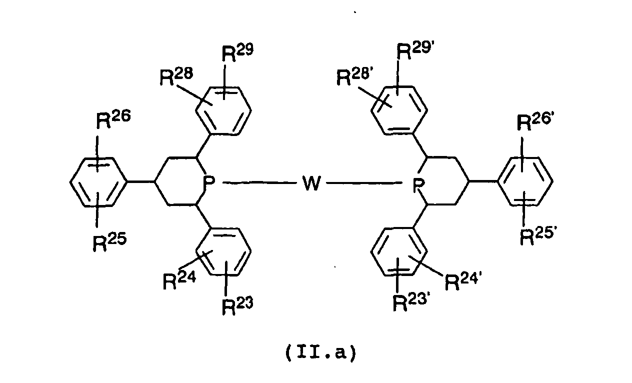 dans lesquelles
R représente un groupe alkyle en C₁-C₂₀, cycloalkyle, aryle en C₆-C₁₂, W' (CHR'CH₂O)ₓR', W'((CH₂)₄O)ₓR' ou un radical de polymère présentant un poids moléculaire moyen numérique de l'ordre de 500 à 50 000, qui est constitué d'éthylène et/ou de butadiène,
W' représentant une simple liaison ou un groupe alkylène en C₁-C₄,
R' représentant de l'hydrogène ou un groupe alkyle en C₁-C₂₀, et
x représentant un nombre entier de 1 à 240,
R²³, R^{23'}, R²⁴, R^{24'}, R²⁵, R^{25'}, R²⁶, R^{26'}, R²⁸ , R^{28'}, R²⁹, R^{29'}, R³⁰ et R³¹ représentant indépendamment les uns des autres de l'hydrogène ou un groupe alkyle, alcoxy, carboxyle, carboxylate, trifluorométhyle, -SO₃H, sulfonate, NE¹E² ou alkylène-NE¹E², E¹ et E² représentant indépendamment l'un de l'autre de l'hydrogène ou un groupe alkyle ou cycloalkyle,
R²⁷ représente de l'hydrogène ou un groupe alkyle, cycloalkyle, aryle ou aralkyle,
R³², R³³, R³⁴, R³⁵, R³⁶, R³⁷ et R³⁸ représentent indépendamment l'un de l'autre un groupe alkyle ou cycloalkyle,
R" représente de l'hydrogène ou un groupe phényle,
A¹ et A² représentent, conjointement avec les atomes de carbone voisins du phosphacyclohexane sur lesquels ils sont fixés, un système de noyaux condensés avec chaque fois 1 ou 2 autres noyaux,
W représente un pont comportant 1 à 20 atomes de carbone qui peut être interrompu par des hétéroatomes.

9. Procédé suivant la revendication 5, dans lequel le catalyseur mis en oeuvre dans l'étape b) présente, comme ligand, au moins une polyamine qui n'est sensiblement pas soluble dans l'eau, présente un poids moléculaire moyen >1000 daltons et au moins 10 atomes d'azote, de préférence une polyamine dérivée ou une polyéthylènimine qui est constituée essentiellement des unités de la formule (IV) : 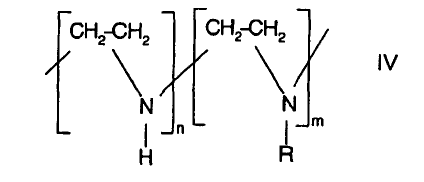 dans laquelle la somme de m+n vaut au moins 10 et le rapport m/m+n est de 0,01 à 1 et R représente des radicaux alkyle, cycloalkyle, aryle, aralkyle, alkylcarbonyle identiques ou différents et comportant jusqu'à 30 atomes de C ou des radicaux hydroxyalkyl(poly)oxyalkylène comportant jusqu'à 500 unités alkylénoxy.

10. Procédé de préparation de mélanges d'alcools fonctionnalisés, dans lequel on soumet un mélange d'alcools suivant l'une des revendications 1 ou 2 à une alcoxylation, une glycosidation, une sulfatation, une phosphatation, une alcoxylation et sulfatation ultérieure ou à une alcoxylation et phosphatation ultérieure.

11. Mélange d'alcools fonctionnalisé, que l'on peut obtenir par un procédé suivant la revendication 10.

12. Utilisation des mélanges d'alcools fonctionnalisés suivant la revendication 11, comme agents tensioactifs, comme ou dans des dispersants, des adjuvants de papier, des adjuvants de matière textile, des adjuvants du cuir, des solvants de saleté, des inhibiteurs de corrosion, des adjuvants pour des dispersions, des inhibiteurs d'incrustation ainsi que dans des produits pour l'industrie de traitement du métal, des produits de processus galvaniques, dans des vernis, des encres d'impression, des compositions de pigment, des formulations phytosanitaires, dans le traitement des eaux et dans l'industrie de traitement des matières synthétiques.
